# EUROPÄISCHE PATENTANMELDUNG

(11) **EP 3 907 272 A1**
(43) Veröffentlichungstag der Anmeldung: **10.11.2021**
(21) Anmeldenummer: 21175950.1
(22) Anmeldetag: 11.03.2016
(51) Int. Cl.: C11D 3/386, C11D 1/02, C11D 1/06, C11D 1/66

(54) **ZUSAMMENSETZUNG ENTHALTEND PEPTIDASE UND BIOTENSID**

(30) Priorität: 18.03.2015 EP 15159546
(62) Teilanmeldung aus: 16709437.4
(71) Anmelder: Evonik Operations GmbH, 45128 Essen (DE)
(72) Erfinder: SCHILLING, Martin, 53121 Bonn (DE); DAVIDS, Ilona, 47647 Kerken (DE); VAN LOGCHEM, Monica Desiree, 4762 PA Zevenbergen (NL); WENK, Hans Henning, 45470 Mülheim an der Ruhr (DE)
(74) Vertreter: Evonik Patent Association

(57) **Zusammenfassung**

Gegenstand der Erfindung sind Zusammensetzungen enthaltend mindestens eine Peptidase ausgewählt aus der Gruppe der Serinproteasen der EC 3.4.21, mindestens ein Biotensid, und gegebenenfalls mindestens ein anionisches Tensid.

## Beschreibung

### Gebiet der Erfindung

Gegenstand der Erfindung sind Zusammensetzungen enthaltend mindestens eine Peptidase und mindestens ein Biotensid, insbesondere ausgewählt aus Rhamnolipiden und Sophorolipiden.

### Stand der Technik

Proteasen werden in Wasch-, Reinigungs- und Spülmitteln eingesetzt und tragen durch den biokatalytischen Abbau zur Auflösung des Schmutzes und damit zur Reinigungsleistung bei. Die Stabilität der Proteasen in den zumeist anionischen Tensidsystemen ist nach wie vor eine Herausforderung für den Formulierer, insbesondere wenn flüssige Formulierungen hergestellt werden sollen, die über einen langen Zeitraum lagerstabil sein und ihre enzymatische Aktivität behalten sollen. Grundsätzlich tragen Tenside zur Denaturierung von Proteinen und somit der Enzymstruktur bei und bewirken damit eine Inaktivierung der Enzymaktivität. Enzymhersteller nutzen die Methoden des Protein Engineerings, um Enzyme mit einer erhöhten Stabilität gegen Tenside zu entwickeln. Allerdings ist dies aufwändig, nicht für alle Enzyme erfolgreich und kann aufgrund der veränderten Proteinstruktur zu einer verringerten spezifischen Aktivität führen.
Alternativ können Enzyme durch den Einsatz milder Tenside stabilisiert werden. So wird etwa Natriumlaurylethersulfat und/oder Fettalkoholethoxylaten zu den als Haupttensid eingesetzten linearen Alkylbenzolsulfonaten zugesetzt, vgl. Kravetz et al. 1985, Lund et al. 2012.
Laut US5156773 können auch Betaine zur Stabilisierung von Proteasen gegenüber anionischen Tensiden eingesetzt werden.
Die DE102007005419 offenbart stickstoffhaltige, aber in einem weiten Bereich nicht-ionische Aminoxide als enzymstabilisierend.

EP0499434, EP2787065 und EP2410039 offenbaren Rhamnolipide und Sophorolipide, alleine oder in Kombination mit anderen anionischen Tensiden, und deren gute Reinigungswirkung auf Wäsche.

Eine gute Reinigungswirkung von Rhamnolipiden in Kombination mit Lipasen wird in den Beispielen von WO2012010406 beschrieben. Hierbei werden keine Formulierungen eingesetzt, die zusätzlich ein weiteres anionisches Tensid enthalten.

Bei der Formulierung von flüssigen Tensidsystemen mit Enzymen, die auch eine Proteaseaktivität enthalten, muss weiterhin darauf geachtet werden, dass die Proteaseaktivität in der Formulierung durch geeignete Additive inhibiert wird, da es ansonsten zum Selbstverdau der Proteasen, bzw. auch zum Verdau anderer Enyzme in der Formulierung kommt. Für diesen Zweck kommen Polyole (z.B. 1,2 Propandiol), Borate und andere Inhibitoren zum Einsatz. Insbesondere die Borate sind in den letzten Jahren aus toxikologischen Gründen in Verruf geraten, und es besteht nach wie vor der Bedarf an einem kostengünstigen, toxikologisch unbedenklichen Ersatz.

### Beschreibung der Erfindung

Überraschenderweise wurde gefunden, dass die Stabilität von Peptidasen insbesondere in anionischen Tensidsystemen durch den Zusatz von Biotensiden, insbesondere Rhamnolipiden und Sophorolipiden, erhöht werden kann. Weiterhin kann die ansonsten benötigte Menge an Proteaseinhibitor reduziert werden, oder es kann gar vollständig auf Proteaseinhibitor verzichtet werden.

Gegenstand der vorliegenden Erfindung sind daher Zusammensetzungen enthaltend
A) mindestens eine Peptidase,
B) mindestens ein Biotensid, und gegebenenfalls
C) mindestens ein anionisches Tensid.

Ein Vorteil der erfindungsgemäßen Zusammensetzung besteht darin, dass der Anteil an in ihr vorhandenen Tensiden, die auf nachwachsenden Rohstoffen basieren, vorzugsweise mehr als 50 Gew.-% bezogen auf die Gesamtmenge der in der Zusammensetzung enthaltenen Tenside beträgt.
Ein weiterer Vorteil der erfindungsgemäßen Zusammensetzung besteht darin, dass als Rohstoffe für die Biotenside Zucker oder Zucker und Glyceride und/oder Fettsäuren verwendet werden können.
Ein weiterer Vorteil der erfindungsgemäßen Zusammensetzung ist, dass diese sehr mild ist.

Noch ein Vorteil der vorliegenden Erfindung ist es, dass in den erfindungsgemäßen Zusammensetzungen die benötigte Menge an Proteaseinhibitor reduziert werden oder gar vollständig auf Proteaseinhibitor verzichtet werden kann.
Ein weiterer Vorteil gegenüber dem Stand der Technik ist die erhöhte Stabilität der Enzyme während des Waschvorgangs, und die damit verbundene verbesserte Reinigungsleistung, zum Beispiel auf Wäsche.
Noch ein Vorteil der vorliegenden Erfindung ist es, dass in den erfindungsgemäßen Zusammensetzungen auch Proteasen eingesetzt werden können, deren Stabilität bisher für den Einsatz in Waschmittelformulierungen nicht ausreichend war.
Noch ein Vorteil der vorliegenden Erfindung ist, dass keine oder weniger Komplexbildner (Builder) eingesetz werden müssen, um eine ausreichende Waschleistung in hartem Wasser zu gewährleisten.

Die erfindungsgemäßen Zusammensetzungen sowie deren Verwendungen werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere bezüglich der in Bezug genommenen Sachverhalte vollständig zum Offenbarungsgehalt der vorliegenden Erfindung gehören. Wenn im nachfolgenden Mittelwerte angegeben sind, so handelt es sich, wenn nicht anders angegeben, um zahlengemittelte Mittelwerte. Bei Prozentangaben handelt es sich, wenn nicht anders angegeben um Angaben in Gewichtsprozent. Werden nachfolgend Messwerte angegeben so wurden diese, wenn nicht anders angegeben, bei einer Temperatur von 25 °C und einem Druck von 1013 mbar bestimmt.
Unter dem Begriff "anionisches Tensid" wird im Zusammenhang mit der vorliegenden Erfindung ein Tensid verstanden, bei welchem bei einem pH-Wert von 7 und 20 °C mindestens 90 mol-% der Moleküle mindestens eine negativ geladene Gruppe aufweisen. Bevorzugt weisen sie keinen isolelektrischen Punkt von pH_{IEP}=2-12 bei 25°C auf, wobei in einer wässrigen 10 millimolaren Kaliumchloridlösung als Hintergrundelektrolyt gemessen wird.
Unter dem Begriff "Stabilisierung einer enzymatischen Aktivität" wird im Zusammenhang mit der vorliegenden Erfindung insbesondere verstanden, dass das betrachtete Enzyme, wenn so lange über einen Zeitraum bei 30 °C gelagert, dass ein Aktivitätsverlust eintritt, weniger Aktivität in Anwesenheit des Stabilisators, vorliegend das Biotensid, verliert, verglichen zu der verlorenen Aktivität unter sonst gleichen Bedingungen unter der Abwesenheit des Stabilisators.

Die erfindungsgemäße Zusammensetzung enthält als Komponente A) mindestens eine Peptidase. Peptidasen sind Enzyme der Enzymklasse ("EC") 3.4.
Bevorzugt sind die enthaltenen Peptidasen ausgewählt aus der Gruppe der Proteasen. Als Proteasen sind alle bekannten Proteasen des Standes der Technik, inklusive der chemisch oder genetisch veränderten Proteasen, geeignet. Hierzu gehören insbesondere Serinproteasen der EC 3.4.21 und Metalloproteasen der EC 3.4.24. Bevorzugte enthaltene Peptidasen sind die Trypsine und die Chymotrypsin ähnlichen Proteasen der EC 3.4.21.1, EC 3.4.21.2 und EC 3.4.21.4 und ganz besonders bevorzugt die Subtilisine der EC 3.4.21.62. Besonders bevorzugt enthaltene Metalloproteasen sind ausgewählt aus der Gruppe der Thermolysine der EC 3.4.24.27 und der Bacillolysine der EC 3.4.24.28 .
Beispiele kommerziell verfügbarer Proteasen umfassen Kannase™, Everlase™, Esperase™, Alcalase™, Neutrase™, Durazym™, Savinase™, Ovozyme™, Liquanase™, Co- ronase™, Polarzyme™, Pyrase™, Pancreatic Trypsin NOVO (PTN), Bio-Feed™ Pro and Clear- Lens™ Pro (alle von Novozymes A/S, Bagsvaerd, Denmark). Weitere kommerziell verfügbare Proteasen umfassen Ronozyme™ Pro, Maxatase™, Maxacal™, Maxapem™, Optic- lean™, Properase™, Purafect™, Purafect Ox™, Purafact Prime™, Excellase™, FN2™, FN 3™ und FN4™ (Genencor International Inc., Gist-Brocades, BASF, DSM). Auch Henkel/ Kemira Proteasen sind geeignet, wie z.B. BLAP (Sequenz in Abbildung 29 von US 5,352,604 mit folgenden Punktmutationen: S99D + S101 R + S103A + V104I + G159S, im Folgenden BLAP genannt), BLAP R (BLAP S3T + V4I + V199M + V205I + L217D), BLAP X (BLAP mit folgenden Punktmutationen: S3T + V4I + V205I) und BLAP F49 (BLAP mit folgenden Punktmutationen: S3T + V4I + A194P + V199M + V205I + L217D) und KAP (*Bacillus alkalophilus* Subtilisin mit folgenden Punktmutationen: A230V + S256G + S259N von Kao.

Im Rahmen der vorliegenden Erfindung werden unter Biotensiden alle durch Fermentation hergestellten Glycolipide verstanden.

Als Rohstoffe für die Herstellung der Biotenside können Kohlenhydrate, insbesondere Zucker wie z.B. Glucose und/oder lipophile Kohlenstoff-Quellen wie Fette, Öle, Partialglyceride, Fettsäuren, Fettalkohole, langkettige gesättigte oder ungesättigte Kohlenwasserstoffe eingesetzt werden. Vorzugsweise sind in den erfindungsgemäßen Zusammensetzungen keine Biotenside vorhanden, die nicht durch Fermentation von Glycolipiden hergestellt werden, wie z. B. Lipoproteine.

Bevorzugt enthält die erfindungsgemäße Zusammensetzung als Komponente B) mindestens ein Biotensid Rhamnolipide, Sophorolipide, Glucoselipide, Celluloselipide, Mannosylerythritollipide und/oder Trehaloselipide, bevorzugt Rhamnolipide und/oder Sophorolipide. Die Biotenside, insbesondere Glykolipidtenside können z. B. wie in EP 0 499 434, US 7,985 722, WO 03/006146, JP 60-183032, DE 19648439, DE 19600743, JP 01-304034, CN 1337439, JP 2006-274233, KR 2004033376, JP 2006-083238, JP 2006-070231, WO 03/002700, FR 2740779, DE 2939519, US 7,556,654, FR 2855752, EP 1445302, JP 2008-062179 und JP 2007-181789 oder den dort zitierten Schriften hergestellt werden. Geeignete Biotenside können z. B. von der Firma Soliance, France bezogen werden.

Bevorzugt weist die erfindungsgemäße Zusammensetzung als Biotenside Rhamnolipide, insbesondere Mono-, Di- oder Polyrhamnolipide und/oder Sophorolipide auf. Besonders bevorzugt weist die erfindungsgemäße Zusammensetzung eines oder mehrere der in EP 1 445 302 A mit den Formeln (I), (II) bzw. (III) beschriebenen Rhamnolipide bzw. Sophorolipide auf.

Unter dem Begriff "Rhamnolipid" im Zusammenhang mit der vorliegenden Erfindung werden insbesondere Verbindungen der allgemeinen Formel (I) oder deren Salze verstanden, 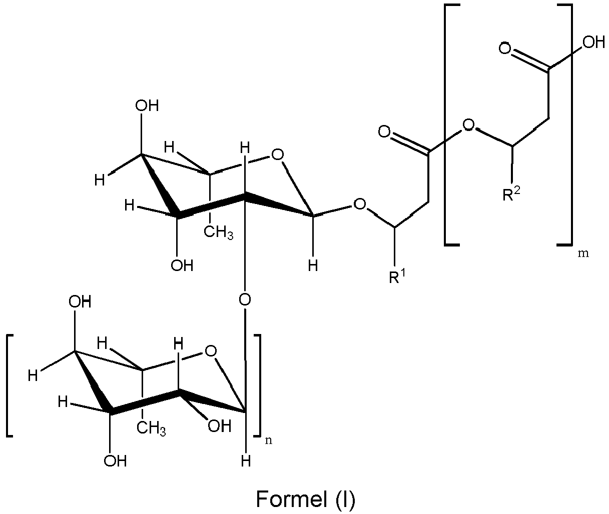 wobei
m = 2, 1 oder 0,
n = 1 oder 0,
R¹ und R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undecenyl und Tridecenyl und (CH₂)ₒ-CH₃ mit o = 1 bis 23, bevorzugt 4 bis 12.
Unter dem Begriff "di-Rhamnolipid" im Zusammenhang mit der vorliegenden Erfindung werden Verbindungen der allgemeinen Formel (I) oder deren Salze verstanden, bei denen n =1.
Unter dem Begriff "mono-Rhamnolipid" im Zusammenhang mit der vorliegenden Erfindung werden Verbindungen der allgemeinen Formel (I) oder deren Salze verstanden, bei denen n =0.
Distinkte Rhamnolipide werden gemäß folgender Nomenklatur abgekürzt:
Unter "diRL-CXCY" werden di-Rhamnolipide der allgemeinen Formel (I) verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.
Unter "monoRL-CXCY" werden mono-Rhamnolipide der allgemeinen Formel (I) verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.

Die verwendete Nomenklatur unterscheidet somit nicht zwischen "CXCY" und "CYCX". Für Rhamnolipide mit m=0 wird entsprechend monoRL-CX bzw. diRL-CX verwendet. Ist einer der oben genannten Indices X und/oder Y mit ":Z" versehen, so bedeutet dies, dass der jeweilige Rest R¹ und/oder R² = ein unverzweigter, unsubstituierter Kohlenwasserstoffrest mit X-3 bzw. Y-3 Kohlenstoffatomen aufweisend Z Doppelbindungen darstellt.
Zur Bestimmung des Gehaltes an Rhamnolipiden im Zusammenhang mit der vorliegenden Erfindung wird lediglich die Masse des Rhamnolipid-Anions, somit "allgemeinen Formel (I) abzüglich ein Wasserstoff" berücksichtigt.
Zur Bestimmung des Gehaltes an Rhamnolipiden im Zusammenhang mit der vorliegenden Erfindung werden alle Rhamnolipide durch Ansäuern in die protonierte Form (vgl. allgemeine Formel (I)) überführt und mittels HPLC quantifiziert.
Die in den erfindungsgemäßen Zusammensetzungen enthaltenen Rhamnolipide liegen aufgrund des gegebenen pH-Wertes mindestens teilweise als Salz vor.
In erfindungsgemäß bevorzugten Zusammensetzungen sind die Kationen der enthaltenen Rhamnolipid-Salze, ausgewählt aus der Gruppe umfassend, bevorzugt bestehend aus, Li⁺, Na⁺, K⁺, Mg²⁺, Ca²⁺, Al³⁺, NH₄⁺, primäre Ammoniumionen, sekundäre Ammoniumionen, tertiäre Ammoniumionen und quaternäre Ammoniumionen.
Beispielhafte Vertreter von geeigneten Ammoniumionen sind Tetramethylammonium, Tetraethylammonium , Tetrapropylammonium, Tetrabutylammonium und [(2-Hydroxyethyl)trimethylammonium] (Cholin) sowie die Kationen von 2-Aminoethanol (Ethanolamin, MEA), Diethanolamin (DEA), 2,2',2"-Nitrilotriethanol (Triethanolamin, TEA), 1-Aminopropan-2-ol (Monoisopropanolamin), Ethylendiamin, Diethylentriamin, Triethylentetramin, Tetraethylenepentamine, 1,4-Diethylendiamin (Piperazin), Aminoethylpiperazin und Aminoethylethanolamin.
Es können auch Mischungen der vorgenannten Kationen als Kationen der enthaltenen Rhamnolipid-Salze erfindungsgemäß vorliegen.
Besonders bevorzugte Kationen, sind ausgewählt aus der Gruppe umfassend, bevorzugt bestehend aus Na⁺, K⁺, NH₄⁺ und das Triethanolammonium-Kation.

Eine erfindungsgemäß bevorzugte Zusammensetzung ist dadurch gekennzeichnet, dass sie eine Mischung von Rhamnolipiden enthält, wobei in der Mischung das Gewichtsverhältnis von di-Rhamnolipiden zu mono-Rhamnolipiden größer als 51:49, bevorzugt größer 75:25, besonders bevorzugt 97:3, insbesondere größer 98:2 ist. Erfindungsgemäß bevorzugte Zusammensetzung ist dadurch gekennzeichnet, dass die Rhamnolipid-Mischung
51 Gew.-% bis 95 Gew.-%, bevorzugt 70 Gew.-% bis 90 Gew.-%, besonders bevorzugt 75 Gew.-% bis 85 Gew.-%, diRL-C10C10 und
0,5 Gew.-% bis 9 Gew.-%, bevorzugt 0,5 Gew.-% bis 3 Gew.-%, besonders bevorzugt 0,5 Gew.-% bis 2 Gew.-%, monoRL-C10C10
enthält,
wobei sich die Gewichtsprozente auf die Summe aller enthaltenen Rhamnolipide beziehen,

Erfindungsgemäß bevorzugte Zusammensetzung ist dadurch gekennzeichnet, dass die Rhamnolipid-Mischung neben den oben genannten diRL-C10C10- und monoRL-C10C10 Gehalten
0,5 Gew.-% bis 15 Gew.-%, bevorzugt 3 Gew.-% bis 12 Gew.-%, besonders bevorzugt 5 Gew.-% bis 10 Gew.-%, diRL-C10C12:1
enthält,
wobei sich die Gewichtsprozente auf die Summe aller enthaltenen Rhamnolipide beziehen.

Erfindungsgemäß bevorzugte Zusammensetzung ist dadurch gekennzeichnet, dass die Rhamnolipid-Mischung neben den oben genannten diRL-C10C10- und monoRL-C10C10 Gehalten
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12 und/oder, bevorzugt und
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12:1,
enthält,
wobei sich die Gewichtsprozente auf die Summe aller enthaltenen Rhamnolipide beziehen.

Es kann vorteilhaft sein und ist daher bevorzugt, wenn die in der erfindungsgemäßen Zusammensetzung enthaltene Rhamnolipid-Mischung neben den oben genannten diRL-C10C10- und monoRL-C10C10 Gehalten
0,1 Gew.-% bis 25 Gew.-%, bevorzugt 2 Gew.-% bis 10 Gew.-%, besonders bevorzugt 4 Gew.-% bis 8 Gew.-%, diRL-C8C10,
enthält,
wobei sich die Gewichtsprozente auf die Summe aller enthaltenen Rhamnolipide beziehen.

Erfindungsgemäß besonders bevorzugte Zusammensetzung ist dadurch gekennzeichnet, dass die Rhamnolipid-Mischung neben den oben genannten diRLC10C10- und monoRL-C10C10 Gehalten
0,5 Gew.-% bis 15 Gew.-%, bevorzugt 3 Gew.-% bis 12 Gew.-%, besonders bevorzugt 5 Gew.-% bis 10 Gew.-%, diRL-C10C12:1,
0,5 bis 25 Gew.-%, bevorzugt 5 Gew.-% bis 15 Gew.-%, besonders bevorzugt 7 Gew.-% bis 12 Gew.-%, diRL-C10C12,
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12 und
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12:1,
enthält,
wobei sich die Gewichtsprozente auf die Summe aller enthaltenen Rhamnolipide beziehen.

Es ist überdies hinaus bevorzugt, wenn die in der erfindungsgemäßen Zusammensetzung enthaltene Rhamnolipid-Mischung Rhamnolipide der Formel monoRL-CX bzw. diRL-CX in nur kleinen Mengen enthält. Insbesondere enthält die erfindungsgemäße Mischungszusammensetzung bevorzugt
0 Gew.-% bis 5 Gew.-%, bevorzugt 0,001 Gew.-% bis 3 Gew.-%, besonders bevorzugt 0,01 Gew.-% bis 1 Gew.-%, diRLC10, wobei sich die Gewichtsprozente auf die Summe aller enthaltenen Rhamnolipide beziehen, und unter dem Begriff "0 Gew.-%" keine nachweisbare Menge zu verstehen ist.

Verfahren zur Herstellung der entsprechenden Rhamnolipid-Mischungen werden zum Beispiel in der EP2786743 und EP2787065 offenbart.

Sophorolipide können erfindungsgemäß in ihrer Säureform oder ihrer Lactonform eingesetzt werden. Zum Begriff "Säureform" von Sophorolipiden wird auf die allgemeine Formel (la) der EP2501813 verwiesen, zum Begriff "Lactonform" von Sophorolipiden wird auf die allgemeine Formel (Ib) der EP2501813 verwiesen.

Zu Bestimmung des Gehaltes an Sophorolipiden in der Säure- bzw. Lactonform in einer Formulierung sei auf die EP 1 411 111 B1, Seite 8, Absatz [0053] verwiesen.

Bevorzugte erfindungsgemäße Formulierungen enthalten als Komponente B) ein Sophorolipid, bei dem das Gewichtsverhältnis von Lactonform zur Säureform in dem Bereich von 20 zu 80 bis 80 zu 20, ganz besonders bevorzugt in den Bereichen von 30 zu 70 bis 40 zu 60, liegt.

Bevorzugt enthält die erfindungsgemäße Zusammensetzung die Komponente C) mindestens ein anionisches Tensid.

Bevorzugt in der erfindungsgemäßen Zusammensetzung enthaltene anionische Tenside sind ausgewählt aus der Gruppe umfassend, bevorzugt bestehend aus, Alkylsulfaten, Alkylethersulfaten, gegebenenfalls alkoxylierte Sulfosuccinaten, gegebenenfalls alkoxylierte Methylsulfosuccinaten, gegebenenfalls alkoxylierte Sulfonaten, gegebenenfalls alkoxylierte Glycinaten, gegebenenfalls alkoxylierte Glutamaten, gegebenenfalls alkoxylierte Isethionaten, gegebenenfalls alkoxylierte Carboxylaten, gegebenenfalls alkoxylierte Anisate, gegebenenfalls alkoxylierte Levulinate, gegebenenfalls alkoxylierte Tartrate, gegebenenfalls alkoxylierte Lactylate, gegebenenfalls alkoxylierte Taurate, gegebenenfalls alkoxylierte Alaninate, gegebenenfalls alkoxylierte Phosphate, gegebenenfalls alkoxylierte Sulfoacetate, gegebenenfalls alkoxylierte Sulfosuccinamate, gegebenenfalls alkoxylierte Sarcosinaten und gegebenenfalls alkoxylierte Phosphonaten.

Bevorzugt in der erfindungsgemäßen Zusammensetzung als anionisches Tensid enthaltene Alkylsulfate oder Alkylethersulfate sind ausgewählt aus der Gruppe bestehend aus C4- bis C24-, bevorzugt C6- bis C18-, besonders bevorzugt C8- bis C14-, Alkylsulfaten und Alkylethersulfaten. Die Alkylreste können linear oder verzweigt sein, wobei lineare bevorzugt sind. Geeignete verzweigte Alkylreste umfassen Methyldecyl Gruppen, Methylundecyl-Gruppen, Methyldodecyl-Gruppen Ethyldecyl-Gruppen, Ethylundecyl-Gruppen und Ethyldodecyl-Gruppen, wie beispielsweise 1-Methyldecyl-, 1-Methylundecyl, 1-Methyldodecyl-, 1-Ethyldecyl, 1-Ethylundecyl und 1-Ethyldodecyl.

Der Zusatz einer Alkyl-oder Alkenylgruppe mit dem Suffix "eth" beschreibt allgemein die Zugabe von einem oder mehreren Ethylenoxid-Einheiten, beispielsweise bezieht sich Trideceth auf eine ethoxylierte Tridecylgruppe, und das Suffix "-n", wobei n eine ganze Zahl ist, die Anzahl von solchen Ethylenoxideinheiten pro Gruppe, beispielsweise "Trideceth-3" bezeichnet eine Gruppe ethoxylierter Tridecylalkohol mit 3 Ethylenoxid-Einheiten pro Tridecylgruppe.
In einer bevorzugten Ausführungsform ist das Alkylsulfat oder Alkylethersulfat ausgewählt aus,
Ammonium C12-15 Alkyl Sulfate, Ammonium C12-16 Alkyl Sulfate, Ammonium Capryleth Sulfate, Ammonium Cocomonoglyceride Sulfate, Ammonium Coco-Sulfate, Ammonium C12-15 Pareth Sulfate, Ammonium Laureth Sulfate, Ammonium Laureth-5 Sulfate, Ammonium Laureth-7 Sulfate, Ammonium Laureth-9 Sulfate, Ammonium Laureth-12 Sulfate, Ammonium Lauryl Sulfate, Ammonium Myreth Sulfate, Ammonium Myristyl Sulfate, Ammonium Nonoxynol-4 Sulfate, Ammonium Nonoxynol-30 Sulfate, Ammonium Palm Kernel Sulfate, Ammonium Trideceth Sulfate, DEA-C12-13 Alkyl Sulfate, DEA-C12-15 Alkyl Sulfate, DEA-Cetyl Sulfate, DEA-C12-13 Pareth-3 Sulfate, DEA-Laureth Sulfate, DEA-Lauryl Sulfate, DEA-Myreth Sulfate, DEA-Myristyl Sulfate, DEA-Trideceth Sulfate, Diethylamine Laureth Sulfate, Magnesium Coceth Sulfate, Magnesium Coco-Sulfate, Magnesium Laureth Sulfate, Magnesium Laureth-5 Sulfate, Magnesium Laureth-8 Sulfate, Magnesium Laureth-16 Sulfate, Magnesium Lauryl Sulfate, Magnesium Myreth Sulfate, Magnesium Oleth Sulfate, Magnesium PEG-3 Cocamide Sulfate, Magnesium/TEA-Coco-Sulfate, MEA-Laureth Sulfate, MEA-Lauryl Sulfate, MEA-Trideceth Sulfate, MIPA C12-15 Pareth Sulfate, MIPA-Laureth Sulfate, MIPA-Lauryl Sulfate, MIPA-Trideceth Sulfate, Mixed Isopropanolamines Lauryl Sulfate, Potassium Laureth Sulfate, Potassium Lauryl Sulfate, Sodium C8-10 Alkyl Sulfate, Sodium C10-16 Alkyl Sulfate, Sodium C11-15 Alkyl Sulfate, Sodium C12-13 Alkyl Sulfate, Sodium C12-15 Alkyl Sulfate, Sodium C12-18 Alkyl Sulfate, Sodium C16-20 Alkyl Sulfate, Sodium Caprylyl Sulfate, Sodium Cetearyl Sulfate, Sodium Cetyl Sulfate, Sodium Cholesteryl Sulfate, Sodium Coceth Sulfate, Sodium Coceth-30 Sulfate, Sodium Coco/Hydrogenated Tallow Sulfate, Sodium Cocomonoglyceride Sulfate, Sodium Coco-Sulfate, Sodium C9-15 Pareth-3 Sulfate, Sodium C10-15 Pareth Sulfate, Sodium C10-16 Pareth-2 Sulfate, Sodium C12-13 Pareth Sulfate, Sodium C12-14 Pareth-3 Sulfate, Sodium C12-15 Pareth Sulfate, Sodium C12-15 Pareth-3 Sulfate, Sodium C13-15 Pareth-3 Sulfate, Sodium C12-14 Sec-Pareth-3 Sulfate, Sodium Deceth Sulfate, Sodium Decyl Sulfate, Sodium Ethylhexyl Sulfate, Sodium Laneth Sulfate, Sodium Laureth Sulfate, Sodium Laureth-5 Sulfate, Sodium Laureth-7 Sulfate, Sodium Laureth-8 Sulfate, Sodium Laureth-12 Sulfate, Sodium Laureth-40 Sulfate, Sodium Lauryl Sulfate, Sodium/MEA-PEG-3 Cocamide Sulfate, Sodium Myreth Sulfate, Sodium Myristyl Sulfate, Sodium Nonoxynol-1 Sulfate, Sodium Nonoxynol-3 Sulfate, Sodium Nonoxynol-4 Sulfate, Sodium Nonoxynol-6 Sulfate, Sodium Nonoxynol-8 Sulfate, Sodium Nonoxynol-10 Sulfate, Sodium Nonoxynol-25 Sulfate, Sodium Octoxynol-2 Sulfate, Sodium Octoxynol-6 Sulfate, Sodium Octoxynol-9 Sulfate, Sodium Oleth Sulfate, Sodium Oleyl Sulfate, Sodium PEG-4 Cocamide Sulfate, Sodium PEG-4 Lauramide Sulfate, Sodium Stearyl Sulfate, Sodium Tallow Sulfate, Sodium/TEA C12-13 Pareth-3 Sulfate, Sodium Trideceth Sulfate, Sodium Tridecyl Sulfate, Sulfated Castor Oil, Sulfated Coconut Oil, Sulfated Glyceryl Oleate, Sulfated Olive Oil, Sulfated Peanut Oil, TEA-C10-15 Alkyl Sulfate, TEA-C11-15 Alkyl Sulfate, TEA-C12-13 Alkyl Sulfate, TEA-C12-14 Alkyl Sulfate, TEA-C12-15 Alkyl Sulfate, TEA-Coco-Sulfate, TEA-C11-15 Pareth Sulfate, TEA-C12-13 Pareth-3 Sulfate, TEA-Laneth-5 Sulfate, TEA-Laureth Sulfate, TEA-Lauryl Sulfate, TEA-Oleyl Sulfate, TEA-PEG-3 Cocamide Sulfate, TEA-Trideceth Sulfate, TIPA-Laureth Sulfate, TIPA-Lauryl Sulfate, wobei Sodium Laureth Sulfate ganz besonders bevorzugt ist.

Bevorzugt in der erfindungsgemäßen Zusammensetzung als anionisches Tensid enthaltene gegebenenfalls alkoxylierte Sulfosuccinate und/oder Methylsulfosuccinate sind ausgewählt aus der Gruppe bestehend aus gegebenenfalls alkoxylierten C4- bis C24-, bevorzugt C6- bis C18-, besonders bevorzugt C8- bis C14-, Sulfosuccinaten und/oder Methylsulfosuccinaten. Die Sulfosuccinate und/oder Methylsulfosuccinate können eine oder zwei Alkylreste enthalten, Monoalkylsulfosuccinate und Monomethylsulfosuccinate sind bevorzugt. Die Alkylreste können linear oder verzweigt sein, wobei lineare bevorzugt sind. Alkoxylierte Sulfosuccinate und/oder Methylsulfosuccinate können insbesondere einen Alkoxylierungsgrad zwischen 1 und 10, besonders bevorzugt zwischen 2 und 5 aufweisen.

Die Alkoxygruppe ist bevorzugt ausgewählt aus ethoxy.

Besonders bevorzugt enthaltene gegebenenfalls alkoxylierte Sulfosuccinate sind ausgewählt aus der Gruppe bestehend aus Disodium Laureth Sulfosuccinate, Disodium C12-14 Pareth-1 Sulfosuccinate, Disodium C12-14 Pareth-2 Sulfosuccinate, Disodium C12-14 Sec-pareth-12 Sulfosuccinate, Disodium C12-14 Sec-pareth-3 Sulfosuccinate, Disodium C12-14 Sec-pareth-5 Sulfosuccinate, Disodium C12-14 Sec-pareth-7 Sulfosuccinate, Disodium C12-14 Sec-pareth-9 Sulfosuccinate, Disodium C12-14 Pareth Sulfosuccinate, Di-Triethanolamine Oleamido PEG-2 Sulfosuccinate, Disodium Oleamido PEG-2 Sulfosuccinate, Disodium Cocamido Monoisopropanolamine PEG-4 Sulfosuccinate, Disodium Cocamido PEG-4 Sulfosuccinate, Disodium Coceth-3 Sulfosuccinate, Disodium Cocoyl Butyl Gluceth-10 Sulfosuccinate, Disodium Deceth-5 Sulfosuccinate, Disodium Deceth-6 Sulfosuccinate, Disodium Laneth-5 Sulfosuccinate, Disodium Lauramido PEG-2 Sulfosuccinate, Disodium Lauramido PEG-5 Sulfosuccinate, Disodium Laureth Sulfosuccinate, Disodium Laureth-12 Sulfosuccinate, Disodium Laureth-6 Sulfosuccinate, Disodium Laureth-9 Sulfosuccinate, Disodium Oleamido PEG-2 Sulfosuccinate, Disodium Oleth-3 Sulfosucciante, Disodium Palmitamido PEG-2 Sulfosuccinate, Disodium PEG-5 Laurylcitrate Sulfosuccinate, Disodium PEG-8 Palm Glycerides Sulfosuccinate, Disodium Sitostereth-14 Sulfosuccinate, Disodium Undecylenamide PEG-2 Sulfosuccinate, Magnesium Laureth-3 Sulfosuccinate, Monoethanolamine Laureth-2 sulfosuccinate, Diammonium C12-14 Pareth-1 Sulfosuccinate, Diammonium C12-14 Pareth-2 Sulfosuccinate, Diammonium C12-14 Sec-pareth-12 Sulfosuccinate, Diammonium C12-14 Sec-pareth-3 Sulfosuccinate, Diammonium C12-14 Sec-pareth-5 Sulfosuccinate, Diammonium C12-14 Sec-pareth-7 Sulfosuccinate, Diammonium C12-14 Sec-pareth-9 Sulfosuccinate, Diammonium C12-14 Pareth Sulfosuccinate, Di-Triethanolamine Oleamido PEG-2 Sulfosuccinate, Diammonium Oleamido PEG-2 Sulfosuccinate, Diammonium Cocamido Monoisopropanolamine PEG-4 Sulfoscuccinate, Diammonium Cocamido PEG-4 Sulfoscuccinate, Diammonium Coceth-3 Sulfosucciante, Diammonium Cocoyl Butyl Gluceth-10 Sulfosuccinate, Diammonium Deceth-5 Sulfosuccinate, Diammonium Deceth-6 Sulfosuccinate, Diammonium Laneth-5 Sulfosuccinate, Diammonium Lauramido PEG-2 Sulfosuccinate, Diammonium Lauramido PEG-5 Sulfosuccinate, Diammonium Laureth Sulfosuccinate, Diammonium Laureth-12 Sulfosuccinate, Diammonium Laureth-6 Sulfosuccinate, Diammonium Laureth-9 Sulfosuccinate, Diammonium Oleamido PEG-2 Sulfosuccinate, Diammonium Oleth-3 Sulfosucciante, Diammonium Palmitamido PEG-2 Sulfosuccinate, Diammonium PEG-5 Laurylcitrate Sulfosuccinate, Disodium PEG-8 Palm Glycerides Sulfosuccinate, Diammonium Sitostereth-14 Sulfosuccinate, Diammonium Undecylenamide PEG-2 Sulfosuccinate, Ammonium Lauryl Sulfosuccinate, Diammonium Lauramido-MEA Sulfosuccinate, Diammonium Lauryl Sulfosuccinate, Dipotassium Lauryl Sulfosuccinate, Disodium Babassuamido MEA-Sulfosuccinate, Disodium Cetearyl Sulfosuccinate, Disodium Cetyl Sulfosuccinate, Disodium Cocamido MEA-Sulfosuccinate, Disodium Cocamido MIPA-Sulfosuccinate, Disodium Coco-Glucoside Sulfosuccinate, Disodium Coco-Sulfosuccinate, Disodium Hydrogenated Cottonseed Glyceride Sulfosuccinate, Disodium Isodecyl Sulfosuccinate, Disodium Isostearamido MEA-Sulfosuccinate, Disodium Isostearamido MIPA-Sulfosuccinate, Disodium Isostearyl Sulfosuccinate, Disodium Lauramido MEA-Sulfosuccinate, Disodium Lauramido MIPA Glycol Sulfosuccinate, Disodium Lauryl Sulfosuccinate, Disodium Myristamido MEA-Sulfosuccinate, Disodium Oleamido MEA-Sulfosuccinate, Disodium Oleamido MIPA-Sulfosuccinate, Disodium Oleyl Sulfosuccinate, Disodium Polyglyceryl-3 Caprate/Caprylate Sulfosuccinate, Disodium Ricinoleamido MEA-Sulfosuccinate,
Disodium Stearamido MEA-Sulfosuccinate, Disodium Stearyl Sulfosuccinate, Disodium Tallamido MEA-Sulfosuccinate, Disodium Tallowamido MEA-Sulfosuccinate, Disodium Tridecylsulfosuccinate, Disodium Undecylenamido MEA-Sulfosuccinate, Diethylhexyl Sodium Sulfosuccinate, Dinonyl Sodium Sulfosuccinate, Diisononyl Sodium Sulfosuccinate, Dioctyl Sodium Sulfosuccinate, Diheptyl Sodium Sulfosuccinate, Dihexyl Sodium Sulfosuccinate, Dineopentyl Sodium Sulfosuccinate, Diisoamyl Sodium Sulfosuccinate, Dipentyl Sodium Sulfosuccinate, Diamyl Sodium Sulfosuccinate, Dibutyl Sodium Sulfosuccinate, Diisobutyl Sodium Sulfosuccinate, Dicapryl Sodium Sulfosuccinate, Didecyl Sodium Sulfosuccinate, Diundecyl Sodium Sulfosuccinate, Dilauryl Sodium Sulfosuccinate, Dicocoyl Sodium Sulfosuccinate, Ditridecyl Sodium Sulfosuccinate, Dipropylheptyl Sodium Sulfosuccinate, Dicyclohexyl Sodium Sulfosuccinate, Ammonium Diethylhexyl Sulfosuccinate, Ammonium Dinonyl Sulfosuccinate, Ammonium Diisononyl Sulfosuccinate, Ammonium Dioctyl Sodium Sulfosuccinate, Ammonium Diheptyl Sulfosuccinate, Ammonium Dihexyl Sulfosuccinate, Ammonium Dineopentyl Sulfosuccinate, Ammonium Diisoamyl Sulfosuccinate, Ammonium Dipentyl Sulfosuccinate, Ammonium Diamyl Sulfosuccinate, Ammonium Dibutyl Sulfosuccinate, Ammonium Diisobutyl Sulfosuccinate, Ammonium Dicapryl Sulfosuccinate, Ammonium Didecyl Sulfosuccinate, Ammonium Diundecyl Sulfosuccinate, Ammonium Dilauryl Sulfosuccinate, Ammonium Dicocoyl Sulfosuccinate, Ammonium Ditridecyl Sulfosuccinate, Ammonium Dipropylheptyl Sulfosuccinate, Ammonium Dicyclohexyl Sulfosuccinate, Diethylhexyl Potassium Sulfosuccinate, Dinonyl Potassium Sulfosuccinate, Diisononyl Potassium Sulfosuccinate, Dioctyl Potassium Sulfosuccinate, Diheptyl Potassium Sulfosuccinate, Dihexyl Potassium Sulfosuccinate, Dineopentyl Potassium Sulfosuccinate, Diisoamyl Potassium Sulfosuccinate, Dipentyl Potassium Sulfosuccinate, Diamyl Potassium Sulfosuccinate, Dibutyl Potassium Sulfosuccinate, Diisobutyl Potassium Sulfosuccinate, Dicapryl Potassium Sulfosuccinate, Didecyl Potassium Sulfosuccinate, Diundecyl Potassium Sulfosuccinate, Dilauryl Potassium Sulfosuccinate, Dicocoyl Potassium Sulfosuccinate, Ditridecyl Potassium Sulfosuccinate, Dipropylheptyl Potassium Sulfosuccinate, Dicyclohexyl Potassium Sulfosuccinate, Diethylhexyl Sodium Methylsulfosuccinate, Dinonyl Sodium Methylsulfosuccinate, Diisononyl Sodium Methylsulfosuccinate, Dioctyl Sodium Methylsulfosuccinate, Diheptyl Sodium Methylsulfosuccinate, Dihexyl Sodium Methylsulfosuccinate, Dineopentyl Sodium Methylsulfosuccinate, Diisoamyl Sodium Methylsulfosuccinate, Dipentyl Sodium Methylsulfosuccinate, Diamyl Sodium Methylsulfosuccinate, Dibutyl Sodium Methylsulfosuccinate, Diisobutyl Sodium Methylsulfosuccinate, Dicapryl Sodium Methylsulfosuccinate, Didecyl Sodium Methylsulfosuccinate, Diundecyl Sodium Methylsulfosuccinate, Dilauryl Sodium Methylsulfosuccinate, Dicocoyl Sodium Methylsulfosuccinate, Ditridecyl Sodium Methylsulfosuccinate, Dipropylheptyl Sodium Methylsulfosuccinate, Dicyclohexyl Sodium Methylsulfosuccinate wobei Disodium Laureth Sulfosuccinate ganz besonders bevorzugt ist.

Bevorzugt in der erfindungsgemäßen Zusammensetzung als anionisches Tensid enthaltene gegebenenfalls alkoxylierte Sulfonate sind ausgewählt aus der Gruppe bestehend aus
Sodium C14-16 Olefin Sulfonate, Sodium C12-15 Pareth-15 Sulfonate, Sodium C14-17 Alkyl sec. Sulfonate, Sodium C14 Olefin Sulfonate, Ammonium Cumenesulfonate, Ammonium Dodecylbenzenesulfonate, Calcium Dodecylbenzenesulfonate, DEA-Dodecylbenzenesulfonate, DEA-Methyl Myristate Sulfonate, Disodium Decyl Phenyl Ether Disulfonate, Disodium Lauriminobishydroxypropylsulfonate, Disodium Lauryl Phenyl Ether Disulfonate, Isopropylamine Dodecylbenzenesulfonate, Magnesium Isododecylbenzenesulfonate, Magnesium Lauryl Hydroxypropyl Sulfonate, MEA-C10-13 Alkyl Benzenesulfonate, MIPA-Dodecylbenzenesulfonate, Potassium Dodecylbenzenesulfonate, Potassium Lauryl Hydroxypropyl Sulfonate, Sodium C13-17 Alkane Sulfonate, Sodium C14-18 Alkane Sulfonate, Sodium C10-13 Alkyl Benzenesulfonate, Sodium C9-22 Alkyl Sec Sulfonate, Sodium C14-17 Alkyl Sec Sulfonate, Sodium Caproylethylformyl Benzenesulfonate, Sodium Caprylyl PG-Sulfonate, Sodium Caprylyl Sulfonate, Sodium Cocoglucosides Hydroxypropylsulfonate, Sodium Cocoglyceryl Ether Sulfonate, Sodium Cocomonoglyceride Sulfonate, Sodium C12-14 Olefin Sulfonate, Sodium C14-16 Olefin Sulfonate, Sodium C14-18 Olefin Sulfonate, Sodium C16-18 Olefin Sulfonate, Sodium C14-15 Pareth-PG Sulfonate, Sodium C12-15 Pareth-3 Sulfonate, Sodium C12-15 Pareth-7 Sulfonate, Sodium C12-15 Pareth-15 Sulfonate, Sodium Decylbenzenesulfonate, Sodium Decylglucosides Hydroxypropylsulfonate, Sodium Dodecylbenzenesulfonate, Sodium Hydroxypropyl Palm Kernelate Sulfonate,
Sodium Lauroyl Hydroxypropyl Sulfonate, Sodium Laurylglucosides Hydroxypropylsulfonate, Sodium Methyl Laurate Sulfonate, Sodium Methyl Myristate Sulfonate, Sodium Methyl Palmitate Sulfonate, Sodium Methyl Stearate Sulfonate, Sodium Palm Glyceride Sulfonate, Sodium Phenylnonanoate Sulfonate, Sodium Tridecylbenzenesulfonate, TEA C14-17 Alkyl Sec Sulfonate, TEA-Dodecylbenzenesulfonate, TEA-Tridecylbenzenesulfonate.

Bevorzugt in der erfindungsgemäßen Zusammensetzung als anionisches Tensid enthaltene gegebenenfalls alkoxylierte Glycinate sind ausgewählt aus der Gruppe bestehend aus
Sodium Cocoyl Glycinate, Potassium Cocoyl Glycinate, Sodium Lauroyl Glycinate, Sodium Lauryl Diethylenediaminoglycinate, TEA-Cocoyl Glycinate.

Bevorzugt in der erfindungsgemäßen Zusammensetzung als anionisches Tensid enthaltene gegebenenfalls alkoxylierte Glutamate sind ausgewählt aus der Gruppe bestehend aus
Sodium Cocoyl Glutamate, Disodium Cocoyl Glutamate, Sodium Lauroyl Glutamate, Sodium Cocoyl Hydrolyzed Wheat Protein Glutamate, Dipotassium Capryloyl Glutamate, Dipotassium Undecylenoyl Glutamate, Disodium Capryloyl Glutamate, Disodium Cocoyl Glutamate, Disodium Hydrogenated Tallow Glutamate, Disodium Lauroyl Glutamate, Disodium Stearoyl Glutamate, Disodium Undecylenoyl Glutamate, Potassium Capryloyl Glutamate, Potassium Cocoyl Glutamate, Potassium Lauroyl Glutamate, Potassium Myristoyl Glutamate, Potassium Stearoyl Glutamate, Potassium Undecylenoyl Glutamate, Sodium Capryloyl Glutamate, Sodium Cocoyl Glutamate, Sodium Cocoyl/Hydrogenated Tallow Glutamate, Sodium Cocoyl Hydrolyzed Wheat Protein Glutamate, Sodium Cocoyl/Palmoyl/Sunfloweroyl Glutamate, Sodium Hydrogenated Tallowoyl Glutamate, Sodium Lauroyl Glutamate, Sodium Myristoyl Glutamate, Sodium Olivoyl Glutamate, Sodium Palmoyl Glutamate, Sodium Stearoyl Glutamate, Sodium Undecylenoyl Glutamate, TEA-Cocoyl Glutamate, TEA-Hydrogenated Tallowoyl Glutamate, TEA-Lauroyl Glutamate.

Bevorzugt in der erfindungsgemäßen Zusammensetzung als anionisches Tensid enthaltene gegebenenfalls alkoxylierte Isethionate sind ausgewählt aus der Gruppe bestehend aus

Sodium Lauroyl Methyl Isethionate, Sodium Cocoyl Isethionate, Ammonium Cocoyl Isethionate, Sodium Cocoyl Isethionate, Sodium Hydrogenated Cocoyl Methyl Isethionate, Sodium Lauroyl Isethionate, Sodium Lauroyl Methyl Isethionate, Sodium Myristoyl Isethionate, Sodium Oleoyl Isethionate, Sodium Oleyl Methyl Isethionate, Sodium Palm Kerneloyl Isethionate, Sodium Stearoyl Methyl Isethionate.

Bevorzugt in der erfindungsgemäßen Zusammensetzung als anionisches Tensid enthaltene gegebenenfalls alkoxylierte Carboxylate sind ausgewählt aus der Gruppe bestehend aus
C12-C22 gesättigten und ungesättigten Fettsäuren und ihren Salzen, sowie Trideceth-7 Carboxylic Acid, Sodium Laureth-13 Carboxylate, Sodium Laureth-4 Carboxylate, Laureth-11 Carboxylic Acid, Laureth-5 Carboxylic Acid, Sodium Laureth-5 Carboxylate, Ammonium Laureth-6 Carboxylate, Ammonium Laureth-8 Carboxylate, Capryleth-4 Carboxylic Acid, Capryleth-6 Carboxylic Acid, Capryleth-9 Carboxylic Acid, Ceteareth-25 Carboxylic Acid, Cetyl C12-15 Pareth-8 Carboxylate, Cetyl C12-15-Pareth-9 Carboxylate, Cetyl PPG-2 Isodeceth-7 Carboxylate, Coceth-7 Carboxylic Acid, C9-11 Pareth-6 Carboxylic Acid, C9-11 Pareth-8 Carboxylic Acid, C11-15 Pareth-7 Carboxylic Acid, C12-13 Pareth-5 Carboxylic Acid, C12-13 Pareth-7 Carboxylic Acid, C12-13 Pareth-8 Carboxylic Acid, C12-13 Pareth-12 Carboxylic Acid, C12-15 Pareth-7 Carboxylic Acid, C12-15 Pareth-8 Carboxylic Acid, C12-15 Pareth-12 Carboxylic Acid, C14-15 Pareth-8 Carboxylic Acid, Deceth-7 Carboxylic Acid, Ethylhexeth-3 Carboxylic Acid, Hexeth-4 Carboxylic Acid, Isopropyl C12-15-Pareth-9 Carboxylate, Isopropyl PPG-2 Isodeceth-7 Carboxylate, Isosteareth-6 Carboxylic Acid, Isosteareth-11 Carboxylic Acid, Laureth-3 Carboxylic Acid, Laureth-4 Carboxylic Acid, Laureth-5 Carboxylic Acid, Laureth-6 Carboxylic Acid, Laureth-8 Carboxylic Acid, Laureth-10 Carboxylic Acid, Laureth-11 Carboxylic Acid, Laureth-12 Carboxylic Acid, Laureth-13 Carboxylic Acid, Laureth-14 Carboxylic Acid, Laureth-17 Carboxylic Acid, Magnesium Laureth-11 Carboxylate, MEA-Laureth-6 Carboxylate, MEA PPG-6 Laureth-7 Carboxylate, MEA-PPG-8-Steareth-7 Carboxylate, Myreth-3 Carboxylic Acid, Myreth-5 Carboxylic Acid, Oleth-3 Carboxylic Acid, Oleth-6 Carboxylic Acid, Oleth-10 Carboxylic Acid, PEG-2 Stearamide Carboxylic Acid, PEG-9 Stearamide Carboxylic Acid, Potassium Laureth-3 Carboxylate, Potassium Laureth-4 Carboxylate, Potassium Laureth-5 Carboxylate, Potassium Laureth-6 Carboxylate, Potassium Laureth-10 Carboxylate, Potassium Trideceth-3 Carboxylate, Potassium Trideceth-4 Carboxylate, Potassium Trideceth-7 Carboxylate, Potassium Trideceth-15 Carboxylate, Potassium Trideceth-19 Carboxylate, PPG-3-Deceth-2 Carboxylic Acid, Propyl C12-15 Pareth-8 Carboxylate, Sodium Capryleth-2 Carboxylate, Sodium Capryleth-9 Carboxylate, Sodium Ceteareth-13 Carboxylate, Sodium Ceteth-13 Carboxylate, Sodium C9-11 Pareth-6 Carboxylate, Sodium C11-15 Pareth-7 Carboxylate, Sodium C12-13 Pareth-5 Carboxylate, Sodium C12-13 Pareth-8 Carboxylate, Sodium C12-13 Pareth-12 Carboxylate, Sodium C12-15 Pareth-6 Carboxylate, Sodium C12-15 Pareth-7 Carboxylate, Sodium C12-15 Pareth-8 Carboxylate, Sodium C12-15 Pareth-12 Carboxylate, Sodium C14-15 Pareth-8 Carboxylate, Sodium C12-14 Sec-Pareth-8 Carboxylate, Sodium Deceth-2 Carboxylate, Sodium Hexeth-4 Carboxylate, Sodium Isosteareth-6 Carboxylate, Sodium Isosteareth-11 Carboxylate, Sodium Laureth-3 Carboxylate, Sodium Laureth-4 Carboxylate, Sodium Laureth-5 Carboxylate,
Sodium Laureth-6 Carboxylate, Sodium Laureth-8 Carboxylate, Sodium Laureth-11 Carboxylate, Sodium Laureth-12 Carboxylate, Sodium Laureth-13 Carboxylate, Sodium Laureth-14 Carboxylate, Sodium Laureth-16 Carboxylate, Sodium Laureth-17 Carboxylate, Sodium Lauryl Glucose Carboxylate, Sodium Lauryl Glycol Carboxylate, Sodium PEG-6 Cocamide Carboxylate, Sodium PEG-8 Cocamide Carboxylate, Sodium PEG-3 Lauramide Carboxylate, Sodium PEG-4 Lauramide Carboxylate, Sodium PEG-7 Olive Oil Carboxylate, Sodium PEG-8 Palm Glycerides Carboxylate, Sodium Trideceth-3 Carboxylate, Sodium Trideceth-4 Carboxylate, Sodium Trideceth-6 Carboxylate, Sodium Trideceth-7 Carboxylate, Sodium Trideceth-8 Carboxylate, Sodium Trideceth-12 Carboxylate, Sodium Trideceth-15 Carboxylate, Sodium Trideceth-19 Carboxylate, Sodium Undeceth-5 Carboxylate, Trideceth-3 Carboxylic Acid, Trideceth-4 Carboxylic Acid, Trideceth-7 Carboxylic Acid, Trideceth-8 Carboxylic Acid, Trideceth-15 Carboxylic Acid, Trideceth-19 Carboxylic Acid und Undeceth-5 Carboxylic Acid.

Bevorzugt in der erfindungsgemäßen Zusammensetzung als anionisches Tensid enthaltene gegebenenfalls alkoxylierte Sarcosinate sind ausgewählt aus der Gruppe bestehend aus
Sodium Lauroyl Sarcosinate, Sodium Cocoyl Sarcosinate, Sodium Myristoyl Sarcosinate, TEA-Cocoyl Sarcosinate, Ammonium Cocoyl Sarcosinate, Ammonium Lauroyl Sarcosinate, Dimer Dilinoleyl Bis-Lauroylglutamate/Lauroylsarcosinate, Disodium Lauroamphodiacetate Lauroyl Sarcosinate, Isopropyl Lauroyl Sarcosinate, Potassium Cocoyl Sarcosinate, Potassium Lauroyl Sarcosinate, Sodium Cocoyl Sarcosinate, Sodium Lauroyl Sarcosinate, Sodium Myristoyl Sarcosinate, Sodium Oleoyl Sarcosinate, Sodium Palmitoyl Sarcosinate, TEA-Cocoyl Sarcosinate, TEA-Lauroyl Sarcosinate, TEA-Oleoyl Sarcosinate, TEA-Palm Kernel Sarcosinate.

Weitere Substanzen, die in der erfindungsgemäßen Zusammensetzung als anionisches Tensid enthalten sein können, sind ausgewählt aus der Gruppe bestehend aus Sodium Anisate, Sodium Levulinate, Sodium Coco-Glucoside Tartrate, Sodium Lauroyl Lactylate, Sodium Methyl Cocoyl Taurate, Sodium Methyl Lauroyl Taurate, Sodium Methyl Oleoyl Taurate, Sodium Cocoyl Alaninate, Sodium Laureth-4 Phosphate, Laureth-1 Phosphate, Laureth-3 Phosphate, Potassium Laureth-1 Phosphate, Sodium Lauryl Sulfoacetate und Sodium Coco Sulfoacetate, Disodium Stearyl Sulfosuccinamate, Disodium Tallow Sulfosuccinamate, Tetrasodium Dicarboxyethyl Stearyl Sulfosuccinamate, sowie deren alkoxylierte Varianten und Mischungen davon.

Besonders bevorzugt enthaltene anionische Tenside sind insbesondere die vorgenannten gegebenenfalls alkoxylierte Sulfonate, Alkylsulfate und Alkylethersulfate.

Kommen die Zusammensetzungen in Waschmitteln zum Einsatz, können weitere Inhaltsstoffe enthalten sein, die die anwendungstechnischen und/oder ästhetischen Eigenschaften der Waschmittelformulierung weiter verbessern. Insbesondere zählen hierzu nicht-ionische Tenside, wie z.B. Fettalkoholethoxylate, Aminoxide und Alkylpolyglucoside (APGs), sowie zwitterionische Tenside, wie z.B. Betaine, die die Stabilität der Enzyme weiter erhöhen können. Weiterhin zählen hierzu Stoffe aus der Gruppe der Gerüststoffe, Bleichmittel, Bleichaktivatoren, Parfüme, Parfümträger, Fluoreszenzmittel, Farbstoffe, Schauminhibitoren, Silikonöle, Antiredepositionsmittel, optischen Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell-und Schiebefestmittel, neutrale Füllsalze sowie UV-Absorber.

Beispiele für Gerüststoffe, Builder, Bleichmittel, Bleichaktivatoren, Bleichkatalysatoren werden in der WO 2007/115872, Seite 22, Zeile 7 bis Seite 25, Zeile 26 beschrieben, deren diesbezüglicher expliziter Offenbarungsgehalt durch diese Bezugnahme Teil dieser Offenbarung wird. Antiredepositionsmittel, optischen Aufheller, Vergrauungsinhibitoren, Farbübertragungsinhibitoren werden beispielhaft in der WO 2007/115872 auf Seite 26, Zeile 15 bis Seite 28, Zeile 2 beschrieben, deren diesbezüglicher expliziter Offenbarungsgehalt durch diese Bezugnahme Teil dieser Offenbarung wird. Beispiele von Knitterschutzmittel, antimikrobiellen Wirkstoffe, Germizide, Fungizide, Antioxidantien, Konservierungsmittel, Antistatika, Bügelhilfsmittel, UV-Absorber werden in der WO 2007/115872 auf den Seiten 28, Zeile 14 bis Seite 30, Zeile 22 beispielhaft beschrieben, deren diesbezüglicher expliziter Offenbarungsgehalt durch diese Bezugnahme Teil dieser Offenbarung wird.

Die Zusammensetzungen können optional weitere Enzyme enthalten, die ebenfalls durch die Glycolipide stabilisiert werden, z.B. (Poly)Esterasen, Lipasen bzw. lipolytisch wirkende Enzyme, Amylasen, Cellulasen bzw. andere Glykosylhydrolasen, Hemicellulase, Cutinasen, β-Glucanasen, Oxidasen, Peroxidasen, Mannanasen, Perhydrolasen, Oxireduktasen und/oder Laccasen.

Die erfindungsgemäßen Zusammensetzungen zeichnen sich bevorzugt dadurch aus, dass der Anteil der Summe aller Tenside in den erfindungsgemäßen Zusammensetzungen von 0,1 bis 50 Gew.-%, vorzugsweise 1 bis 25 Gew.-%, bevorzugt 5 bis 20 Gew.-% und besonders bevorzugt 10 bis 20 Gew.-% beträgt, wobei sich die Gewichtsprozente auf die gesamte Zusammensetzung beziehen.
Der Anteil an Biotensid am Gesamttensid beträgt bevorzugt von 5 Gew.-% bis 99 Gew.-%, bevorzugt von 20 Gew.-% bis 95 Gew.-%, besonders bevorzugt von 25 Gew.-% bis 80 Gew.-%, bezogen auf die Gesamttensidmenge in der erfindungsgemäßen Zusammensetzung.

Bevorzugte erfindungsgemäße Zusammensetzungen enthalten als eine Komponente D) Wasser.
In einer bevorzugten Ausführungsform enthält die erfindungsgemäße Zusammensetzung Wasser in einer Menge von 0,001 Gew.-% bis 5 Gew.-%, vorzugsweise 0,01 Gew.-% bis 3 Gew.-%, bevorzugt 0,1 Gew.-% bis 2 Gew.-% Wasser. Diese Ausführungsform deckt beispielsweise lagerstabile Trockenreinigungsmittel, zum Beispiel in Pulver-, Granulat- oder Tablettenform, ab. In einer alternativen bevorzugten Ausführungsform enthält die erfindungsgemäße Zusammensetzung Wasser in einer Menge von 10 Gew.-% bis 95 Gew.-%, vorzugsweise 20 Gew.-% bis 9 0Gew.-%, bevorzugt 30 Gew.-% bis 80 Gew.-% Wasser. Diese alternative Ausführungsform deckt beispielsweise lagerstabile Flüssigreinigungsmittel ab.

Die erfindungsgemäßen Zusammensetzungen weisen bevorzugt einen pH-Wert von 4 bis 12,5 bevorzugt von 5 bis 10, besonders bevorzugt von 5,5 bis 9,0, auf.
Werden die erfindungsgemäßen Zusammensetzungen in z.B. Wäschewaschmittel eingesetzt, so weisen sie bevorzugt einen pH-Wert von 7 bis 12,5 bevorzugt von 7,5 bis 12, besonders bevorzugt von 8 bis 12 auf.
Werden die erfindungsgemäßen Zusammensetzungen in z.B. Handgeschirrspülmittel eingesetzt, so weisen sie alternativ bevorzugt einen pH-Wert von 4 bis 8 bevorzugt von 4,5 bis 7,5, besonders bevorzugt von 5 bis 6,5 auf.
Der "pH-Wert" im Zusammenhang mit der vorliegenden Erfindung ist definiert als der Wert, welcher für entsprechenden Stoff bei 25 °C nach fünf Minuten Rühren mit einer gemäß ISO 4319 (1977) kalibrierten pH-Elektrode gemessen wird.

Bevorzugte erfindungsgemäße Zusammensetzungen enthalten als eine Komponente E) mindestens einen Proteaseinhibtor.
Bevorzugt enthaltene Proteaseinhibitoren sind ausgewählt aus der Liste der reversiblen Proteaseinhibitoren.
Als reversible Proteaseinhibitoren werden häufig Benzamidin-Hydrochlorid, Borax, Borsäuren, Boronsäuren oder deren Salze oder Ester eingesetzt, darunter vor allem Derivate mit aromatischen Gruppen, etwa ortho-, meta- oder para-substituierte Phenylboronsäuren, insbesondere 4-Formylphenyl-Boronsäure, beziehungsweise die Salze oder Ester der genannten Verbindungen. Auch Peptidaldehyde, das heißt Oligopeptide mit reduziertem C-Terminus, insbesondere solche aus 2 bis 50 Monomeren werden zu diesem Zweck eingesetzt. Zu den peptidischen reversiblen Proteaseinhibitoren gehören unter anderem Ovomucoid und Leupeptin. Auch spezifische, reversible Peptid-Inhibitoren für die Protease Subtilisin sowie Fusionsproteine aus Proteasen und spezifischen Peptid-Inhibitoren sind hierfür geeignet.
Besonders bevorzugt wird als Komponente E) Borsäure und/oder ihre Salze eingesetzt.
Es ist erfindungsgemäß bevorzugt, dass, wenn als Komponente E) Borsäure und/oder ihre Salze enthalten ist, zusätzlich Polyole enthalten sind. Diese stabilisieren in Wechselwirkung mit der Borsäure und/oder ihren Salzen sowie den Biotensiden die Peptidaseaktivität in der Formulierung zusätzlich. Bevorzugt eingesetzte Polyole sind 1,2-Propanediol, Ethyleneglycol, Erythritan, Glycerol, Sorbitol, Mannitol, Glucose, Fructose, und Lactose. Das Gewichtsverhältnis von Borsäure und/oder ihren Salzen zu Polyolen liegt erfindungsgemäß in einem Bereich von 1 zu 0,1 bis 1 zu 10, bevorzugt von 1 zu 0,3 bis 1 zu 5.

Besonders bevorzugte erfindungsgemäße Zusammensetzungen enthalten
A) mindestens eine Peptidase,
B) mindestens ein Biotensid,
C) mindestens ein anionisches Tensid,
D) Wasser, und
E) mindestens einen Proteaseinhibtor,
wobei
die Petidase ausgewählt ist aus der Gruppe der Bacillolysine der EC 3.4.24.28, der Gruppe der Thermolysine der EC 3.4.24.27 und der Gruppe der Subtilisine der EC 3.4.21.62,
das Biotensid ausgewählt ist aus der Gruppe umfassend Rhamnolipide und Sophorolipide,
das anionische Tensid ausgewählt ist aus der Gruppe umfassend gegebenenfalls alkoxylierte Sulfonate, der Gruppe der Alkylsulfate und der Gruppe der Alkylethersulfate,
der Proteaseinhibtor ausgewählt ist aus der Gruppe umfassend Borsäure und ihre Salze,
und die Komponenten enthalten sind in einer Menge bezogen auf die Gesamtzusammensetzung von
A) von 0,001 Gew.-% bis 10 Gew.-%, vorzugsweise 0,05 Gew.-% bis 5 Gew.-%, bevorzugt 0,1 Gew.-% bis 3 Gew.-%
B) von 0,5 Gew.-% bis 50 Gew.-%, vorzugsweise 2 Gew.-% bis 40 Gew.-%, bevorzugt 5 Gew.-% bis 30 Gew.-%
C) von 0,1 Gew.-% bis 40 Gew.-%, vorzugsweise 1 Gew.-% bis 35 Gew.-%, bevorzugt 2 Gew.-% bis 30 Gew.-%
D) von 0,001 Gew.-% bis 95 Gew.-%, vorzugsweise 1 Gew.-% bis 90 Gew.-%, bevorzugt 10 Gew.-% bis 60 Gew.-%
E) von 0,001 Gew.-% bis 10 Gew.-%, vorzugsweise 0,01 Gew.-% bis 5 Gew.-%, bevorzugt 0,1 Gew.-% bis 3 Gew.-%.

Ein weiterer Gegenstand der vorliegenden Erfindung ist ein Verfahren zur Stabilisierung von Peptidasen, umfassend die Verfahrensschritte:
a) Bereitstellen mindestens einer Peptidase und
b) Hinzufügen mindestens eines Biotensides,
unter Erhalt einer petidase-stabilisierten Zusammensetzung.

Das erfindungsgemäße Verfahren wird bevorzugt derart durchgeführt, dass als peptidase-stabilisierte Zusammensetzungen erfindungsgemäß bevorzugte, erfindungsgemäße Zusammensetzungen erhalten werden.

Ein weiterer Gegenstand der vorliegenden Erfindung ist die Verwendung von mindestens einem Biotensid zur Stabilisierung der enzymatischen Aktivität von mindestens einer Peptidase, insbesondere in den erfindungsgemäßen Zusammensetzungen.
Bei der erfindungsgemäßen Verwendung werden insbesondere bevorzugte Ausführungsformen der erfindungsgemäßen Zusammensetzungen und deren bevorzugten Komponenten in den bevorzugten Mengen eingesetzt.
Somit ist eine erfindungsgemäß besonders bevorzugte Verwendung dadurch gekennzeichnet, dass die Petidase ausgewählt ist aus der Gruppe umfassend Bacillolysine der EC 3.4.24.28, Subtilisine der EC 3.4.21.62 und Thermolysine der EC 3.4.24.27, und das verwendete Biotensid ausgewählt ist aus der Gruppe umfassend Rhamnolipide und Sophorolipide.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beispiele:

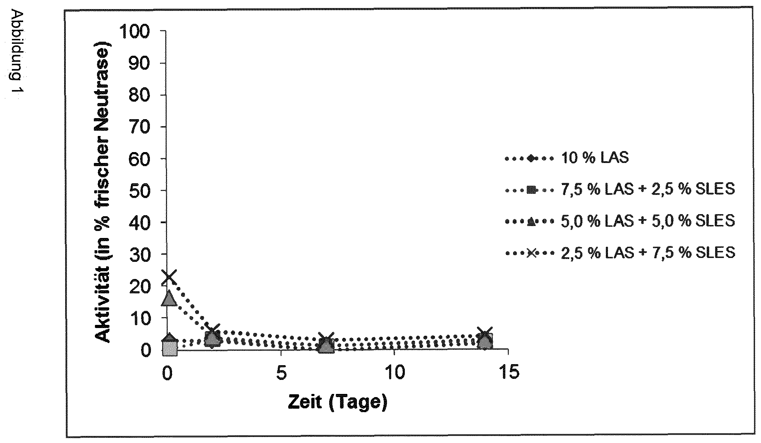

Abbildung 1: Einfluss der Natriumlaurylethersulfat (SLES) Zugabe auf die Lagerstabilität von Neutrase® 0.8 L in einer Formulierung mit linearem Alkylbenzolsulfonat (LAS) (vgl. Tabelle 1). Angabe der Aktivität im Vergleich zu im Kühlschrank gelagertem Enzym. SLES trägt kaum zur Stabilisierung des Enzyms bei.

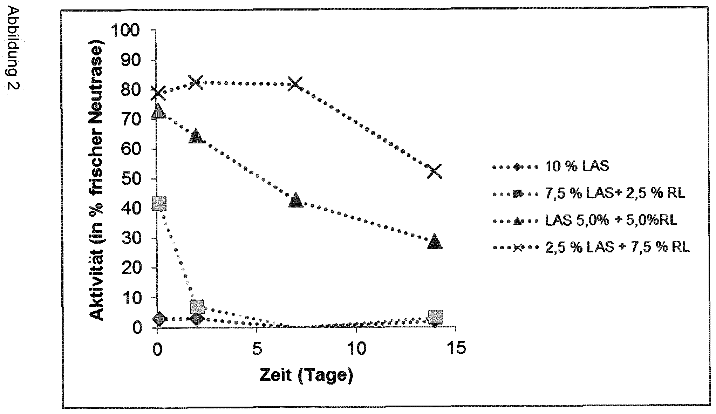

Abbildung 2: Einfluss der Rhamnolipid (RL) Zugabe auf die Lagerstabilität von Neutrase® 0.8 L in einer Formulierung mit LAS (vgl. Tabelle 1). Angabe der Aktivität im Vergleich zu im Kühlschrank gelagertem Enzym. Durch die Rhamnolipidzugabe, kann die Stabiliät des Enzyms drastisch erhöht werden.

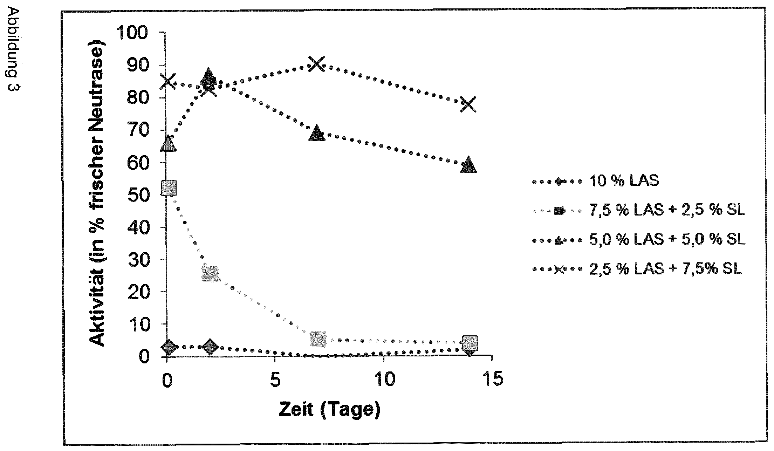

Abbildung 3: Einfluss der Sophorolipid (SL) Zugabe auf die Lagerstabilität von Neutrase® 0.8 L in einer Formulierung mit LAS (vgl. Tabelle 1). Angabe der Aktivität im Vergleich zu im Kühlschrank gelagertem Enzym. Durch die Sophorolipidzugabe, kann die Stabiliät des Enzyms drastisch erhöht werden.

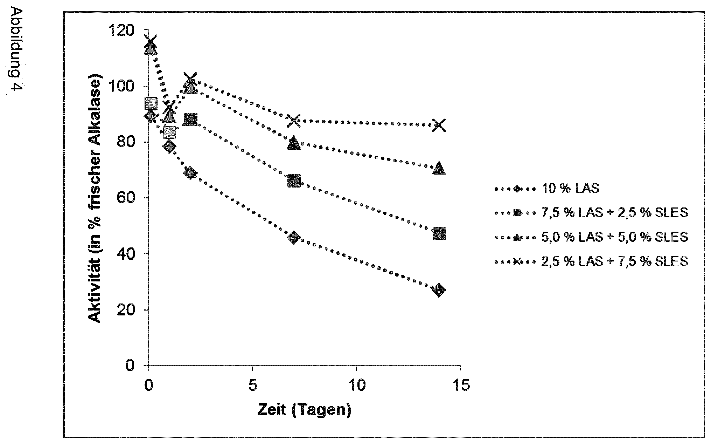

Abbildung 4: Einfluss der Natriumlaurylethersulfat (SLES) Zugabe auf die Lagerstabilität von Alcalase® 2.4 L FG in einer Formulierung mit LAS (vgl. Tabelle 2). Angabe der Aktivität im Vergleich zu im Kühlschrank gelagertem Enzym. SLES trägt deutlich zur Stabilisierung des Enzyms bei.

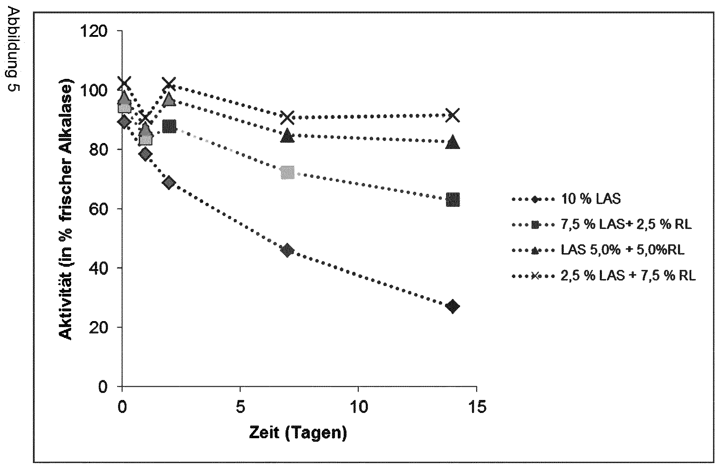

Abbildung 5: Einfluss der Rhamnolipid (RL) Zugabe auf die Lagerstabilität von Alcalase® 2.4 L FG in einer Formulierung mit LAS (vgl. Tabelle 2). Angabe der Aktivität im Vergleich zu im Kühlschrank gelagertem Enzym. Die Stabilisierung des Enzyms durch das Rhamnolipid ist noch wirksamer als bei der Zugabe von SLES (Vgl. Abbildung 4).

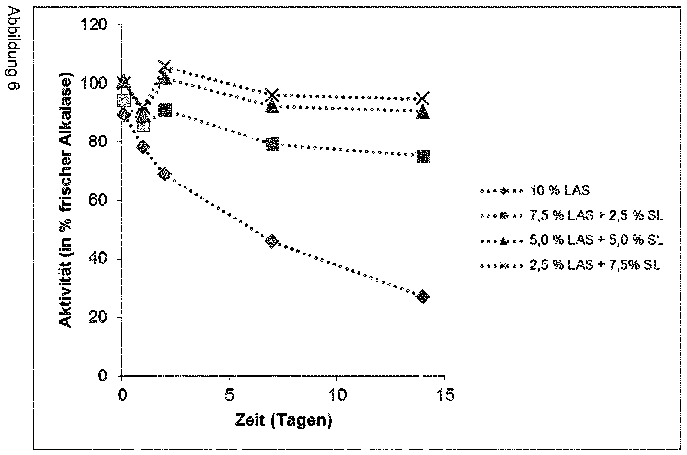

Abbildung 6: Einfluss der Sophorolipid (SL) Zugabe auf die Lagerstabilität von Alcalase® 2.4 L FG in einer Formulierung mit LAS (vgl. Tabelle 2). Angabe der Aktivität im Vergleich zu im Kühlschrank gelagertem Enzym. Die Stabilisierung des Enzyms durch das Sophorolipid ist noch wirksamer als bei der Zugabe von SLES (Vgl. Abbildung 4).

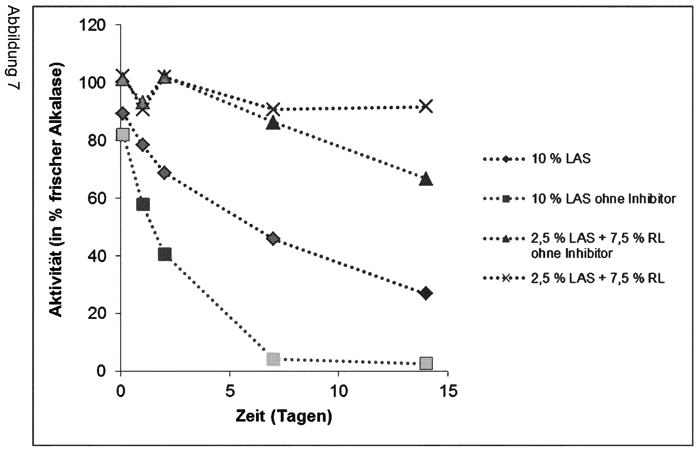

Abbildung 7: Lagerstabilität der Protease Alcalase® 2.4 L FG in Gegenwart und in Abwesenheit von Inhibitoren in einer Formulierung mit LAS und in einer Formulierung mit LAS mit Rhamnolipid (vgl. Tabelle 3). Ohne Inhibitor kommt es im System mit nur LAS zu einem vollständigen Selbstverdau der Protease. In Gegenwart des Rhamnolipids ist der Aktivitätsabfall auch ohne Inhibitor deutlich geringer.

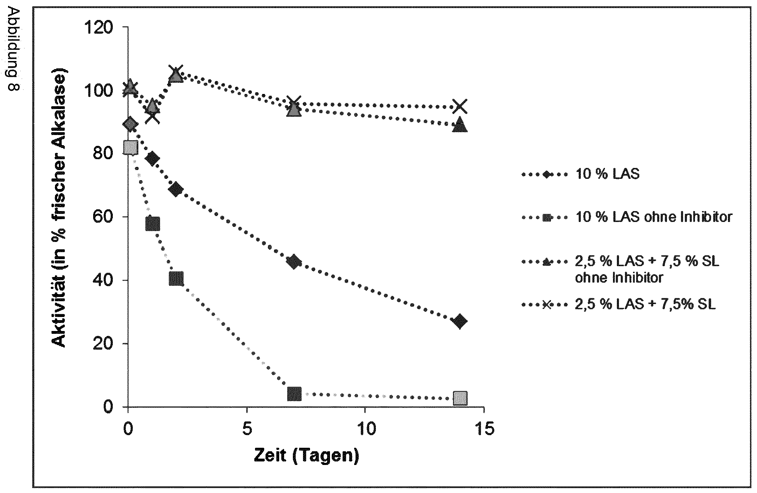

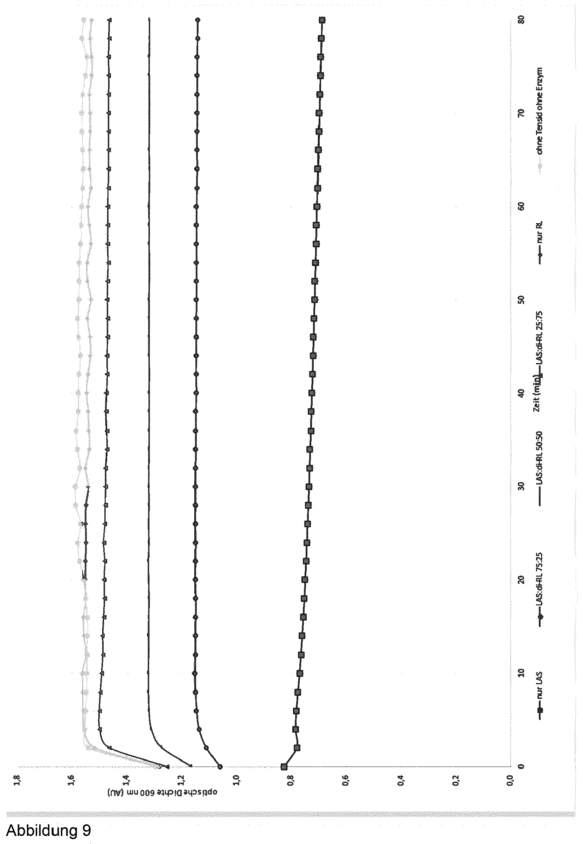

Abbildung 8: Lagerstabilität der Protease Alcalase® 2.4 L FG in Gegenwart und in Abwesenheit von Inhibitoren in einer Formulierung mit LAS und in einer Formulierung mit LAS mit Sophorolipid (vgl. Tabelle 3). Ohne Inhibitor kommt es im System mit nur LAS zu einem vollständigen Selbstverdau der Protease. In Gegenwart des Sophorolipids ist auch ohne Inhibitor kaum ein Aktivitätsabfall zu beobachten. Abbildung 9: Zu Beispiel 4. Einfluss von LAS, RL und Mischungen der beiden Tenside auf die Solubilisierung von Zein. Messungen der optischen Dichte bei 600 nm über die Zeit im Vergleich zu Zein ohne Tensid. Je geringer die optische Dichte, desto höher der Anteil an solubilisiertem Zein. Die Gesamttensidkonzentration betrug immer 0,05 Gew.-%. Die Gewichtsanteile von LAS und RL betrugen hierbei 100:0, 75:25, 50:50, 25:75 und 0:100.

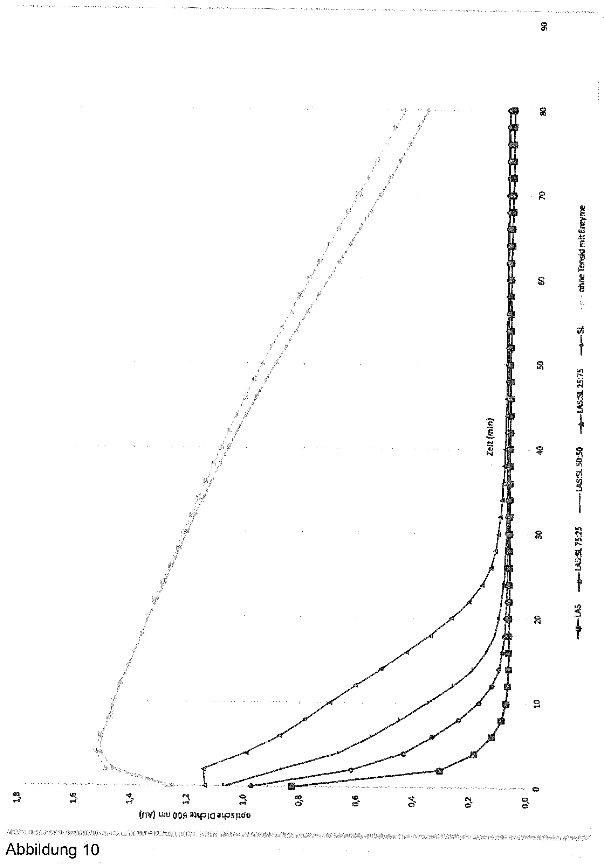

Abbildung 10: Zu Beispiel 4. Einfluss von LAS, SL und Mischungen der beiden Tenside auf die Solubilisierung von Zein. Messungen der optischen Dichte bei 600 nm über die Zeit im Vergleich zu Zein ohne Tensid. Je geringer die optische Dichte, desto höher der Anteil an solubilisiertem Zein. Die Gesamttensidkonzentration betrug immer 0,05 Gew.-%. Die Gewichtsanteile von LAS und SL betrugen hierbei 100:0, 75:25, 50:50, 25:75 und 0:100.

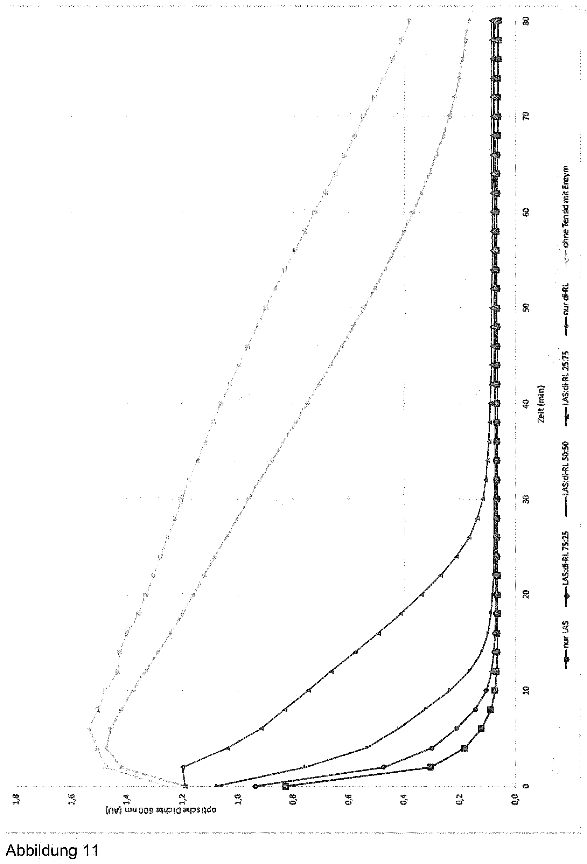

Abbildung 11: Zu Beispiel 4. Einfluss von LAS, RL und Mischungen der beiden Tenside auf die Solubilisierung von Zein in Kombination mit einer Protease. Messungen der optischen Dichte bei 600 nm über die Zeit im Vergleich zur enzymatischen Solubilisierung von Zein ohne Tensid. Je geringer die optische Dichte, desto höher der Anteil an solubilisiertem Zein. Die Gesamttensidkonzentration betrug immer 0,05 Gew.-%. Die Gewichtsanteile von LAS zu RL betrugen hierbei 100:0, 75:25, 50:50, 25:75 und 0:100.

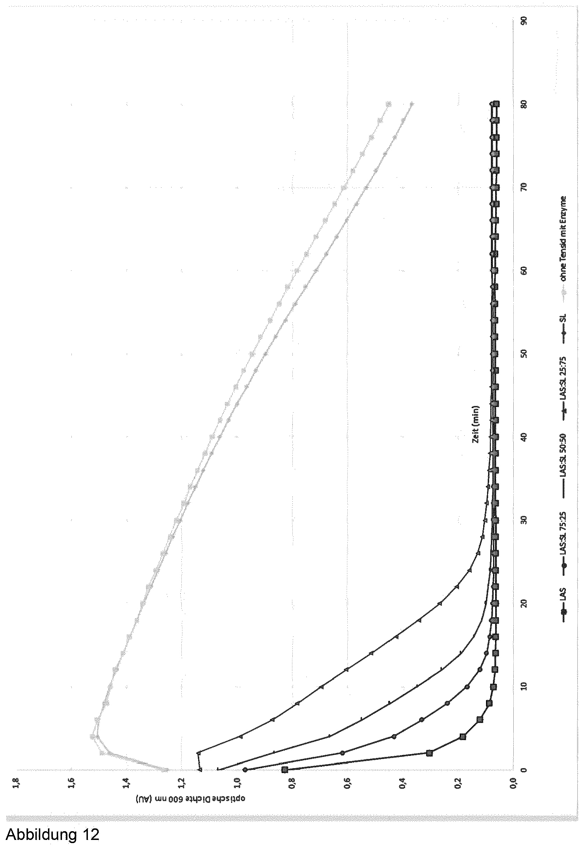

Abbildung 12: Zu Beispiel 4. Einfluss von LAS, SL und Mischungen der beiden Tenside auf die Solubilisierung von Zein in Kombination mit einer Protease. Messungen der optischen Dichte bei 600 nm über die Zeit im Vergleich zur enzymatischen Solubilisierung von Zein ohne Tensid. Je geringer die optische Dichte, desto höher der Anteil an solubilisiertem Zein. Die Gesamttensidkonzentration betrug immer 0,05 Gew.-%. Die Gewichtsanteile von LAS zu SL betrugen hierbei 100:0, 75:25, 50:50, 25:75 und 0:100.

### Beispiele:

### Beispiel 1: Vergleich der Lagerstabilität einer Protease in linearem Alkylbenzolsulfonat (LAS), Mischungen von LAS mit Natriumlaurylethersulfat (SLES), Mischungen von LAS mit Rhamnolipid und Mischungen von LAS mit Sophorolipid

Die Untersuchungen sollten die stabilisierende Wirkung von SLES, Rhamnolipid und Sophorolipid auf die Proteasen Neutrase und Alcalase in Gegenwart von LAS zeigen. Für Untersuchungen zur Lagerstabilität wurden Tensidgemische in einem 0,1 M Triethanolaminpuffer (TEA), pH = 8 hergestellt. Weiterhin wurden die Proteaseinhibitoren 1,2 Propandiol und Borsäure zugegeben. Bei Bedarf wurden die Mischungen durch Zugabe von Säure oder Lauge auf pH = 8 gestellt. Zu den entsprechenden Mischungen wurden Proteasen aus flüssigen Präparationen hinzugegeben, gemischt und die Gemische bei 30 °C gelagert (vgl. Tabelle 1 und 2). Bei den Proteasen handelte es sich um die Produkte Neutrase® 0.8 L und Alcalase® 2.4 L FG (ein Subtilisin). Aus diesen Zusammensetzungen wurden zu verschiedenen Zeiten Proben entnommen und mit 0,1 M Phosphatpuffer, pH = 7, 100-fach verdünnt. Je 100 µl dieser verdünnten Lösungen wurden in die Kavitäten einer 96-well Mikrotiterplatte pipetiert. Dann wurden 100 µl einer 0,4 mg/ml Lösung des Substrates N-Succinyl-ALA-ALA-Pro-PHE-P-Nitroanilide (Sigma Aldrich) in 0,1 M Phosphatpuffer, pH = 7, dazugegeben, und die Enzymaktivität über die Hydrolyse des Substrates bei 37 °C in einem Mikrotiterplattenreader (Tecan, Infitite® M200 Pro) bei 410 nm gemessen. Die Aktivität wurde aus der Anfangssteigung berechnet und auf die Enzymaktivität des im Kühlschrank gelagerten Enzyms bezogen.

Als Rhamnolipide wurde in allen Beispielen folgende Mischung eingesetzt:
Mittels HPLC nachgewiesene Rhamnolipidspezies waren:

| RL gesamt [%] (HPLC) | **91** |
|---|---|
| diRL-C8C10 | 13.9 |
| monoRL-C8C10 | 0.51 |
| diRL-C10C10 | 61.4 |
| monoRL-C10C10 | 1.4 |
| diRL-C10C12:1 | 5.9 |
| diRL-C10C12 | 5.5 |
| other RL | 2.2 |

Als Sophorolipide wurde in allen Beispielen ein Soporolipid der Firma Ecover eingesetzt, welches ein Säure zu Lacton Verhältnis von 60:40 besitzt.

**Tabelle 1: Zusammensetzungen in Gew.-% der Anteile in 0,1 M TEA Puffer. Der pH Wert der Zusammensetzungen wurde auf pH = 8 eingestellt.**

| Zusammensetzung | 1.1 | 1.2 | 1.3 | 1.4 |
|---|---|---|---|---|
| Lineares Alkylbenzolsulfonat (Marlon ARL, Sassol) | 10 | 7,5 | 5 | 2,5 |
| Rhamnolipid oder Sophorolipid oder SLES | - | 2,5 | 5 | 7,5 |
| 1,2 Propandiol | 2,1 | 2,1 | 2,1 | 2,1 |
| Borsäure | 1,6 | 1,6 | 1,6 | 1,6 |
| Neolone PE | 0,4 | 0,4 | 0,4 | 0,4 |
| Neutrase® 0.8 L | 10 | 10 | 10 | 10 |

**Tabelle 2: Zusammensetzungen aus LAS mit und ohne verschiedenen stabilisierenden Tensiden und Alcalase® 2.4 L FG. Angaben in Gew.-% der Anteile in 0,1 M TEA Puffer. Der pH Wert der Zusammensetzungen wurde auf pH = 8 eingestellt.**

| Zusammensetzung | 2.1 | 2.2 | 2.3 | 2.4 |
|---|---|---|---|---|
| Lineares Alkylbenzolsulfonat (Marlon ARL, Sassol) | 10 | 7,5 | 5 | 2,5 |
| Rhamnolipid oder Sophorolipid oder SLES | - | 2,5 | 5 | 7,5 |
| 1,2 Propandiol | 2,1 | 2,1 | 2,1 | 2,1 |
| Borsäure | 1,6 | 1,6 | 1,6 | 1,6 |
| Neolone PE | 0,4 | 0,4 | 0,4 | 0,4 |
| Alcalase® 2.4 L FG | 0,1 | 0,1 | 0,1 | 0,1 |

### Beispiel 2: Erhöhte Enzymstabilität in Abwesenheit eines Proteaseinhibitors

Die Lagerstabiliätstests und Aktivitätsmessungen wurden analog zu Beispiel 1 durchgeführt. Zusätzlich wurden Zusammensetzungen angesetzt, bei denen die Proteaseinhibitoren 1,2-Propandiol und Borsäure nicht zugegeben wurden.

**Tabelle 3: Zusammensetzungen aus LAS mit und ohne verschiedenen stabilisierenden Tensiden, Polyol, Inhibitor und Alcalase® 2.4 L FG. Angaben in Gew.-% der Anteile in 0,1 M TEA Puffer. Der pH Wert der Zusammensetzungen wurde auf pH = 8 eingestellt.**

| Zusammensetzung | 3.1 | 3.2 | 3.3 | 3.4 |
|---|---|---|---|---|
| Lineares Alkylbenzolsulfonat (Marlon ARL, Sassol) | 10 | 10 | 2,5 | 2,5 |
| Rhamnolipid oder Sophorolipid | - | - | 7,5 | 7,5 |
| 1,2 Propandiol | 2,1 | - | 2,1 | - |
| Borsäure | 1,6 | - | 1,6 | - |
| Neolone PE | 0,4 | 0,4 | 0,4 | 0,4 |
| Alcalase® 2.4 L FG | 0,1 | 0,1 | 0,1 | 0,1 |

### Beispiel 3: Beispielformulierungen

### 3.1 Pulverwaschmittel 1

| | |
|---|---|
| Sophorolipid | 12,0 |
| Lineares Natriumalkylbenzolsulfonat | 5,3 % |
| Fettalkoholethoxylat C12-18 (7 EO) | 2,0 % |
| Natriumsalze von Fettsäuren | 2,1 % |
| Antifoam DC2-4248S | 5,0 % |
| Zeolit 4A | 36,3 % |
| Natriumcarbonat | 14,9 % |
| Natriumsalz Acryl-Maleinsäure-Copolymer (Sokalan CP5) | 3,1 % |
| Natrium Silikat | 3,8 % |
| Carboxymethylcellulose | 1,5 % |
| Dequest 2066 (Phosphonat) | 3,6 % |
| Optische Aufheller | 0,3 % |
| Protease (Savinase 8.0) | 0,5 % |
| Natriumperborat Monohydrate | 1,0 % |
| Natriumsulfat | Rest |

### 3.2 Pulverwaschmittel 2

| | |
|---|---|
| Rhamnolipid | 12,0 |
| Lineares Natriumalkylbenzolsulfonat | 5,3 % |
| Fettalkoholethoxylat C12-18 (7 EO) | 2,0 % |
| Natriumsalze von Fettsäuren | 2,1 % |
| Antifoam DC2-4248S | 5,0 % |
| Zeolit 4A | 36,3 % |
| Natriumcarbonat | 14,9 % |
| Natriumsalz Acryl-Maleinsäure-Copolymer (Sokalan CP5) | 3,1 % |
| Natrium Silikat | 3,8 % |
| Carboxymethylcellulose | 1,5 % |
| Dequest 2066 (Phosphonat) | 3,6 % |
| Optische Aufheller | 0,3 % |
| Protease (Savinase 8.0) | 0,5 % |
| Natriumperborat Monohydrate | 1,0 % |
| Natriumsulfat | Rest |

### 3.3. Flüssigwaschmittel 1

| | |
|---|---|
| Sophorolipid | 6,0 % |
| Lineares Natriumalkylbenzolsulfonat | 4,0 % |
| Fettalkoholethoxylat C12-18 (7 EO) | 5,0 % |
| Fettsäure | 1,0 % |
| Phosphonate | 0,5 % |
| Propandiol | 5,0 % |
| Protease (Alcalase® 2.4 L FG) | 1 % |
| 1,2-Benzisothiazoline-3-on ('BIT', z.B."Proxel") | 100 ppm |
| Natriumhydroxid | --> pH 8,5 |
| VE Wasser | Rest |

### 3.4. Flüssigwaschmittel 2

| | |
|---|---|
| Rhamnolipid | 6,0 % |
| Lineares Natriumalkylbenzolsulfonat | 4,0 % |
| Fettalkoholethoxylat C12-18 (7 EO) | 5,0 % |
| Fettsäure | 1,0 % |
| Phosphonate | 0,5 % |
| Propandiol | 5,0 % |
| Protease (Alcalase® 2.4 L FG) | 1 % |
| 1,2-Benzisothiazoline-3-on ('BIT', z.B."Proxel") | 100 ppm |
| Natriumhydroxid | --> pH 8,5 |
| VE Wasser | Rest |

### 3.5. Flüssigwaschmittelkonzentrat 1

| | |
|---|---|
| Rhamnolipid | 30,0 % |
| Natriumlaurylethersulfat | 10,0 % |
| Lineares Natriumalkylbenzolsulfonat | 5,0 % |
| Phosphonate | 0,5 % |
| Natriummetaborat | 1,0 % |
| Propandiol | 2,0 % |
| Protease (Alcalase® 2.4 L FG) | 1 % |
| Lipase | 1 % |
| Amylase | 1 % |
| Duftstoffe | 0,5 % |
| 1,2-Benzisothiazoline-3-on ('BIT', eg "Proxel") | 100 ppm |
| Natriumhydroxid | --> pH 8,5 |
| VE Wasser | Rest |

### 3.6. Flüssigwaschmittelkonzentrat 2

| | |
|---|---|
| Sophorolipid | 30,0 % |
| Natriumlaurylethersulfat | 10,0 % |
| Lineares Natriumalkylbenzolsulfonat | 5,0 % |
| Phosphonate | 0,5 % |
| Natriummetaborat | 1,0 % |
| Propandiol | 2,0 % |
| Protease (Alcalase® 2.4 L FG) | 1 % |
| Lipase | 1 % |
| Amylase | 1 % |
| Duftstoffe | 0,5 % |
| 1,2-Benzisothiazoline-3-on ('BIT', eg "Proxel") | 100 ppm |
| Natriumhydroxid | --> pH 8,5 |
| VE Wasser | Rest |

### Beispiel 4: Verbesserte Proteinsolublisierung durch Zugabe von anionischem Tensid zu Biotensid.

Neben der ausreichenden Lagerstabilität der Enzyme in flüssigen Waschmittelformulierungen, ist die Aktivität von Peptidasen bzw. Proteasen in der Anwendung von entscheidender Bedeutung. Im Zusammenspiel mit den Tensiden tragen sie zur Solubilisierung von nicht wasserlöslichen Proteinen bzw. nicht wasserlöslichem Proteinschmutz bei.
Es wurden verschiedenen Tensidgemische alleine und in Kombination mit einer Protease eingesetzt, um die Solubilisierung des nicht wasserlöslichen, aber wasserdispergierbaren Modellproteins Zein zu untersuchen. Zein ist eine Mischung von Speicherproteinen aus dem Maiskorn.
Alle Lösungen/Dispersionen wurden in 0,1 M TEA Puffer, pH = 8 angesetzt. Eine Stammdispersion von 0,5 Gew- % Zein (Sigma-Aldrich) wurde durch Beschallen im Ultraschallbad für 30 min hergestellt und auf einem Magnetrührer weitergerührt, um die Zeinpartikel in homogener Dispersion für die Probenahme zu halten. Eine 10 Gew.-% Alcalase® 2.4 L FG Stammlösung wurde ebenfalls hergestellt. Tensidstammlösungen bestehend aus linearem Alkylbenzolsulfonat (LAS, Marlon ARL, Sassol) und Biotensid mit einem Gesamttensidgehalt von 0,11 Gew. % wurden hergestellt. Die Gewichtsanteile von linearem LAS und Biotensid betrugen hierbei 100:0, 75:25, 50:50, 25:75 und 0:100. Tensid-, Enzym- und Zeinstammlösungen wurden in Mikrotiterplatten gemischt (220 µl Gesamtflüssigkeisvolumen) und die Trübung bei 600 nm (OD 600) in einem Mikrotiterplattenreader (Tecan, Infitite® M200 Pro) gemessen. Es wurden folgende Konzentrationen eingestellt: 0,25 Gew. % Zein, 0,05 Gew. % Tensid(-mischung), 0,45 % Alcalase® 2.4 L FG. Zunächst wurden Tensidmischung und Enzym vorgelegt, dann die Reaktion durch Zugabe der Zeindispersion gestartet. Alle Lösungen sowie die Messungen im Mikrotiterplattenreader wurden auf 25 °C temperiert. Die Veränderung der Trübung wurde einmal pro Minute über einen Zeitraum von 80 Minuten gemessen. Zwischen den einzelnen Messungen wurde die Platte für 10 Sekunden im Reader geschüttelt. Die abnehmende Trübung läßt sich auf eine Solubilisierung des Zeins zurückführen.
Die Zugabe der Tenside alleine führte zu einer teilweisen Solubilisierung der Zeindispersion. (Abbildung 9 und 10). Die Zugabe von Protease ohne Tensid führte zu einer langsamen, aber beinahe vollständigen Solubilisierung (Abbildung 10 und 11). Die Zugabe von Biotensid und Protease führte zu einer leicht beschleunigten Solubilisierung im Vergleich zum Enzym ohne Tensid. Mit steigendem LAS (Anteil bezogen auf die Gesamttensidmenge) wurde die Solubilisierung zunehmend beschleunigt. Es kommt zu einer synergistischen Effekt zwischen Protease und Tensid(mischung) bei der Proteinsolubilisierung. Eine Mischung aus LAS und Biotensid ist also optimal, um eine schnelle Protein-Solubilisierung in Kombination mit einer Protease zu erzielen und gleichzeitig eine möglichst hohe Lagerstabilität der Protease in der Tensidmischung zu erhalten. Dieser Zusammenhang wird zusätzlich in Tabelle 4 beschrieben.

**Tabelle 4: Einfluss von verschiedenen LAS:Biotensid Mischungsverhältnissen auf die Lagerstabilität einer Protease und die Solubilisierung von Proteinschmutz durch das Tensidgemisch in Kombination mit einer Protease.**

| LAS:Biotensid Anteil am Gesamttensid | Lagerstabilität der Protease im Tensid(gemisch) | Solubilisierung von Proteinschmutz durch Tensidgemisch und Protease |
|---|---|---|
| 100:0 (nicht erfindungsgemäß) | Schlecht | Sehr schnell |
| 75:25 | Besser | Sehr schnell |
| 50:50 | Gut | Schnell |
| 25:75 | Sehr gut | Schnell |
| 0:100 (nicht erfindungsgemäß) | Sehr gut | Langsam |

## Patentansprüche

1. Zusammensetzung enthaltend
A) mindestens eine Peptidase ausgewählt aus der Gruppe der Serinproteasen der EC 3.4.21,
B) mindestens ein Biotensid, und gegebenenfalls
C) mindestens ein anionisches Tensid.

2. Zusammensetzung gemäß Anspruch 1, **dadurch gekennzeichnet, dass** die Peptidase ausgewählt ist aus der Gruppe umfassend die Trypsine und die Chymotrypsin ähnlichen Proteasen der EC 3.4.21.1, EC 3.4.21.2 und EC 3.4.21.4 und die Subtilisine der EC 3.4.21.62.

3. Zusammensetzung gemäß Anspruch 1 oder 2, **dadurch gekennzeichnet, dass** das Biotensid ausgewählt ist aus der Gruppe umfassend Rhamnolipide und Sophorolipide.

4. Zusammensetzung gemäß mindestens einem der vorherigen Ansprüche, **dadurch gekennzeichnet, dass** das enthaltene anionische Tensid ausgewählt ist aus der Gruppe umfassend, bevorzugt bestehend aus, Alkylsulfaten, Alkylethersulfaten, gegebenenfalls alkoxylierte Sulfosuccinaten, gegebenenfalls alkoxylierte Methylsulfosuccinaten, gegebenenfalls alkoxylierte Sulfonaten, gegebenenfalls alkoxylierte Glycinaten, gegebenenfalls alkoxylierte Glutamaten, gegebenenfalls alkoxylierte Isethionaten, gegebenenfalls alkoxylierte Carboxylaten, gegebenenfalls alkoxylierte Anisate, gegebenenfalls alkoxylierte Levulinate, gegebenenfalls alkoxylierte Tartrate, gegebenenfalls alkoxylierte Lactylate, gegebenenfalls alkoxylierte Taurate, gegebenenfalls alkoxylierte Alaninate, gegebenenfalls alkoxylierte Phosphate, gegebenenfalls alkoxylierte Sulfoacetate, gegebenenfalls alkoxylierte Sulfosuccinamate, gegebenenfalls alkoxylierte Sarcosinaten und gegebenenfalls alkoxylierte Phosphonaten. insbesondere gegebenenfalls alkoxylierte Sulfonate, Alkylsulfate und Alkylethersulfate.

5. Zusammensetzung gemäß mindestens einem der vorherigen Ansprüche, **dadurch gekennzeichnet, dass** der Anteil der Summe aller Tenside von 0,1 Gew.-% bis 50 Gew.-%, vorzugsweise 1 Gew.-% bis 25 Gew.-%, bevorzugt 5 Gew.-% bis 20 Gew.-% und besonders bevorzugt 10 Gew.-% bis 20 Gew.-% beträgt, wobei sich die Gewichtsprozente auf die Gesamtzusammensetzung beziehen.

6. Zusammensetzung gemäß mindestens einem der vorherigen Ansprüche, **dadurch gekennzeichnet, dass** der Anteil an Biotensid am Gesamttensid von 5 Gew.-% bis 99 Gew.-%, bevorzugt von 20 Gew.-% bis 95 Gew.-%, besonders bevorzugt von 25 Gew.-% bis 80 Gew.-%, bezogen auf die Gesamttensidmenge in der Zusammensetzung, beträgt.

7. Zusammensetzung gemäß mindestens einem der vorherigen Ansprüche enthaltend
D) Wasser.

8. Zusammensetzung gemäß mindestens einem der vorherigen Ansprüche enthaltend
E) mindestens einen Proteaseinhibtor.

9. Zusammensetzung gemäß Anspruch 8 als Proteaseinhibtor enthaltend Borsäure und/oder ihre Salze.

10. Verwendung von mindestens einem Biotensid zur Stabilisierung der enzymatischen Aktivität von mindestens einer Peptidase.
